# EUROPEAN PATENT SPECIFICATION

(11) **EP 1 131 297 B1**
(45) Date of publication and mention of the grant of the patent: **14.04.2004**
(21) Application number: 99957309.0
(22) Date of filing: 15.11.1999
(51) Int. Cl.: C07D 223/10, C07D 409/12, C07D 403/12, C07D 401/12, A61K 31/55, A61P 35/00

(54) **CERTAIN SUBSTITUTED CAPROLACTAMS, PHARMACEUTICAL COMPOSITIONS CONTAINING THEM AND THEIR USE IN TREATING TUMORS**
SUBSTITUIERTE CAPROLACTAM-DERIVATE, DIESE ENTHALTENDE PHARMAZEUTISCHE ZUBEREITUNGEN SOWIE IHRE VERWENDUNG ZUR BEHANDLUNG VON TUMOREN
CAPROLACTAMES SUBSTITUES, COMPOSITIONS PHARMACEUTIQUES CONTENANT CES COMPOSES ET LEUR UTILISATION DANS LE TRAITEMENT DES TUMEURS

(30) Priority: 17.11.1998 US 193354
(43) Date of publication of application: 12.09.2001
(73) Proprietor: Novartis AG, 4056 Basel (CH); Novartis Pharma GmbH, 1230 Wien (AT)
(72) Inventor: KINDER, Frederick, Ray, Jr., Morristown, NJ 07960 (US); BAIR, Kenneth, Walter, Mountain Lakes, NJ 07046 (US); JAGOE, Christopher, Turchik, Chatham, NJ 07928 (US); VERSACE, Richard, William, Wanaque, NJ 07465 (US); WATTANASIN, Sompong, Hopatcong, NJ 07843 (US)
(74) Representative: Grubb, Philip William
(86) International application number: PCT/EP1999/008767
(87) International publication number: WO 2000/029382

(56) References cited:
- EP-A- 0 687 673
- QUINOA, EMILIO ET AL: "Bengamides, heterocyclic anthelmintics from a Jaspidae marine sponge" JOURNAL OF ORGANIC CHEMISTRY., vol. 51, no. 23, 1986, pages 4494-4497, XP002129550 AMERICAN CHEMICAL SOCIETY. EASTON., US ISSN: 0022-3263
- S. BUDAVARI (ED.): "The Merck Index, 12th edition" 1996 , MERCK RESEARCH LABORATORIES , WHITEHOUSE STATION, N.J. USA; ISBN 0911910-12-3 XP002129551 cited in the application Monograph 3495: Doxorubicin

## Description

The present invention relates to the area of chemotherapeutic agents and, more particularly, relates to certain substituted caprolactams, pharmaceutical compositions comprising said caprolactams, a method of treating tumors, the use of said caprolactams in the chemotherapy of tumors, and a process for preparing said compounds.

Cancer is a serious health problem throughout the world. As a result, an extensive number of research endeavors has been undertaken in an effort to develop therapies appropriate to the treatment and alleviation of cancer in humans.

In the chemotherapeutic area, research has been conducted to develop anti-tumor agents effective against various types of cancer. Oftentimes, anti-tumor agents which have been developed and found effective against cancer cells are, unfortunately, also toxic to normal cells. This toxicity manifests itself in weight loss, nausea, vomiting, hair loss, fatigue, itching, hallucinations, loss of appetite, etc., upon administration of the anti-tumor agent to a patient in need of cancer chemotherapy.

Furthermore, conventionally used chemotherapeutic agents do not have the effectiveness desired or are not as broadly effective against different types of cancers as desired. As a result, a great need exists for chemotherapeutic agents which are not only more effective against all types of cancer, but which have a higher degree of selectivity for killing cancer cells with no or minimal effect on normal healthy cells. In addition, highly effective and selective anti-tumor agents, in particular, against cancers of the colon, bladder, prostate, stomach, pancreas, breast, lung, liver, brain, testis, ovary, cervix, skin, vulva and small intestine are desired. Moreover, anti-tumor activity against colon, breast, lung and prostate cancers as well as melanomas are particularly desired because of the lack of any particular effective therapy at the present time.

The present invention provides new anti-tumor agents which are effective against a variety of cancer cells. More particularly, the present invention relates to certain substituted caprolactams which exhibit a high degree of selectivity in killing cancer cells. The essence of the instant invention is the finding that certain substituted caprolactams are useful in treating tumors.

The invention relates to caprolactams of formula I: 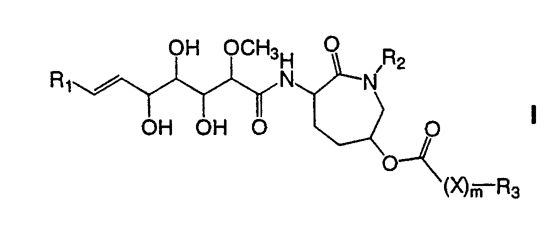 where
- R₁: is (C₁₋₆)alkyl or (C₃₋₆)cycloalkyl;
- R₂: is hydrogen or (C₁₋₆)alkyl;
- X: is (C₁₋₁₂) alkylene; (C₂₋₁₂) alkenylene; or (C₂₋₁₂) alkynylene;
- m: is 0 or 1; and
- R₃: is (C₃₋₈)cycloalkyl; or an aromatic ring system selected from II, III, IV and V: 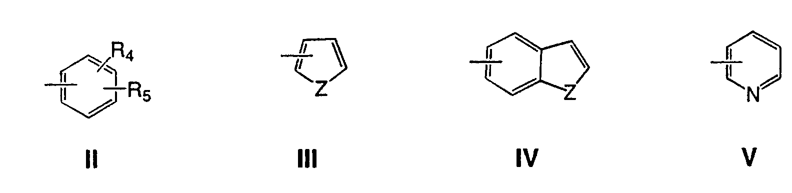
where
- R₄: is hydrogen, chloro, or methoxy;
- R₅: is hydrogen, chloro, (C₁₋₁₈)alkyl or (C₁₋₁₈)alkoxy; and Z is oxygen, sulfur, N-H, or N-CH₃;
or a pharmaceutically acceptable acid addition salt thereof, where possible.

Preferred compounds of formula I are those where
- R₁: is (C₁₋₆) alkyl;
- R₂: is hydrogen or (C₁₋₄) alkyl;
- X: is (C₁₋₆) alkylene or (C₂₋₆) alkenylene;
- m: is 0 or 1; and
- R₃: is (C₃₋₈)cycloalkyl; or an aromatic ring system selected from II, III, IV and V where
R₄ is hydrogen, chloro, or methoxy;
R₅ is hydrogen, chloro, (C₁₋₁₈)alkyl or (C₁₋₁₈)alkoxy; and Z is oxygen, sulfur, N-H, or N-CH₃;
or a pharmaceutically acceptable acid addition salt thereof, where possible.

More preferred compounds are those of formula I where
- R₁: is *i*-propyl or *t*-butyl;
- R₂: is hydrogen or methyl;
- m: is 0 or 1;
- X: is (C₁₋₆) alkylene; and
- R₃: is (C₅₋₇)cycloalkyl; or an aromatic ring system selected from IIa and V: 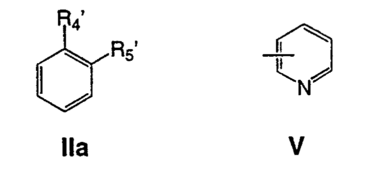 where
R_{4'} is in the *meta* position and is hydrogen or chloro; and
R₅' is in the para position and is hydrogen, chloro, (C₁₋₁₈)alkyl or (C₁₋₁₈)alkoxy;
or a pharmaceutically acceptable acid addition salt thereof, where possible.

Even more preferred compounds are those of formula I where
- R₁: is *i*-propyl or *t*-butyl;
- R₂: is hydrogen or methyl;
- m: is 0 or 1;
- X: is methylene or ethylene; and
- R₃: is (C₅₋₇)cycloalkyl, phenyl, 3,4-dichlorophenyl, 4-methoxyphenyl, 4-*n*-decylphenyl, 4-*n*-decyloxyphenyl or 3-pyridyl;
with the proviso that when m is 0, R₃ is (C₅₋₇)cycloalkyl, 4-*n*-decylphenyl or 4-*n*-decyloxyphenyl;
or a pharmaceutically acceptable acid addition salt thereof, where possible.

In another embodiment, the instant invention provides pharmaceutical compositions, especially for the treatment of tumors in warm-blooded animals, comprising a pharmaceutically acceptable carrier or diluent and a antitumorally effective dose of a compound of formula I above, preferably 3,4,5-trihydroxy-2-methoxy-8,8-dimethy)-*N*-[hexahydro-2-oxo-6-(cyclohexylcarbonyl)oxy-2*H*-azepin-3-yl]non-6-enamide or 3,4,5-trihydroxy-2-methoxy-8,8-dimethyl-*N*-[hexahydro-2-oxo-6-(1-oxo-3-phenylpropoxy)-*2H*-azepin-3-yl]non-6-enamide, or a pharmaceutically acceptable acid addition salt thereof, where possible.

In another embodiment, the instant invention relates to the use of a compound of formula I or of a pharmaceutically acceptable salt of such a compound for the preparation of a pharmaceutical composition for use in the chemotherapy of tumours.

In the above definitions: 1) the alkyl groups containing 1 to 6 carbon atoms are either straight or branched chain, of which examples of the latter include isopropyl, isobutyl, t-butyl, isopentyl, neopentyl, isohexyl, 3-methylpentyl, 2,2-dimethylbutyl, 2,3-dimethylbutyl and 1,1,2,2-tetramethylethyl; and 2) the alkyl and alkoxy groups containing 1 to 18 carbon atoms are either straight or branched chain.

The term "(C₁₋₁₂) alkylene" as used herein refers to a straight or branched chain divalent group consisting solely of carbon and hydrogen and having from 1 to 12 carbon atoms. Examples of "alkylene" groups include methylene, ethylene, propylene, butylene, pentylene, 3-methypentylene, etc.

The term "(C₂₋₁₂) alkenylene" as used herein refers to a straight or branched chain divalent group consisting solely of carbon and hydrogen, containing at least one carbon-carbon double bond, and having from 2 to 12 carbon atoms. Examples of "alkenylene" groups include ethenylene, propenylene, butenylene, pentenylene, 3-methylpentenylene, etc.

The term "(C₂₋₁₂) alkynylene" as used herein refers to a straight or branched chain divalent group consisting solely of carbon and hydrogen, containing at least one carbon-carbon triple bond, and having from 2 to 12 carbon atoms. Examples of "alkynylene" groups include acetylene, propynylene, butynylene, pentynylene, 3-methylpentynylene, etc.

The acid addition salts of the compounds of formula I may be those of pharmaceutically acceptable organic or inorganic acids. Although the preferred acid addition salts are those of hydrochloric and methanesulfonic acid, salts of sulfuric, phosphoric, citric, fumaric, maleic, benzoic, benzenesulfonic, succinic, tartaric, lactic and acetic acid may also be utilized.

The caprolactams of formula I may be prepared as depicted below: 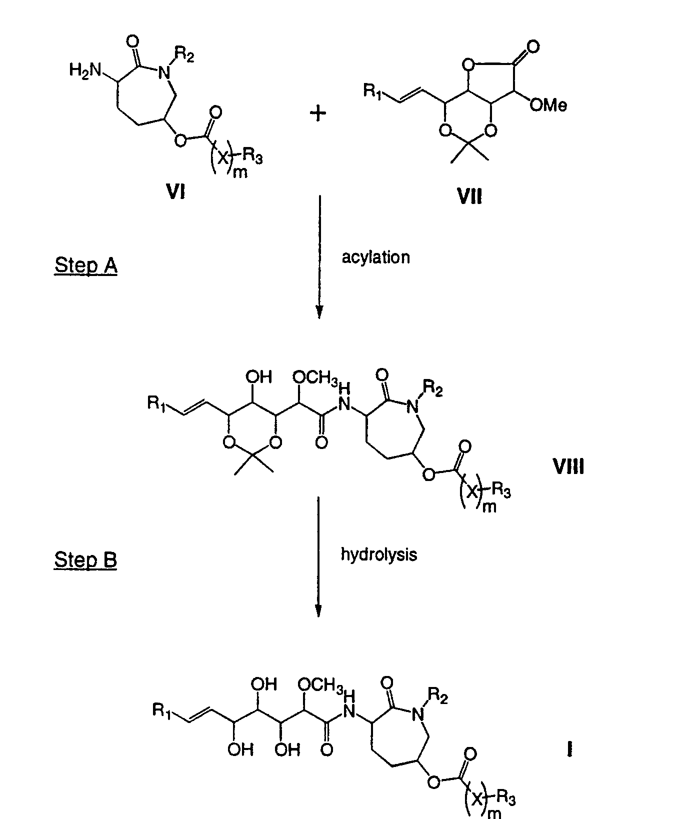 where each R₁, R₂, X, m and R₃ is as defined above.

As to the individual steps, Step A involves the acylation of an aminocaprolactam of formula VI with a lactone compound of formula VII to obtain a diamide compound of formula VIII. The acylation is conducted in a polar, organic solvent, preferably a protic polar solvent such as isopropanol, at a temperature slightly below or at the reflux temperature of the solvent employed for a period of between 4 and 48 hours.

Step B concerns the hydrolysis of the 1,3-dioxane group common to a diamide compound of formula VIII, to obtain a substituted caprolactam compound of formula I. The hydrolysis is typically carried out by dissolving the diamide in a mixture of solvents consisting of 1) a protic acid, preferably an organic acid such as trifluoroacetic acid, 2) a protic solvent, preferably water, and 3) an inert organic solvent, preferably a cyclic ether such as tetrahydrofuran, at a temperature of between 0 °C and 25 °C for a period of between 5 minutes and 2 hours.

Alternatively, the diamide compounds of formula VIII may be prepared according to the following 3-step reaction scheme: 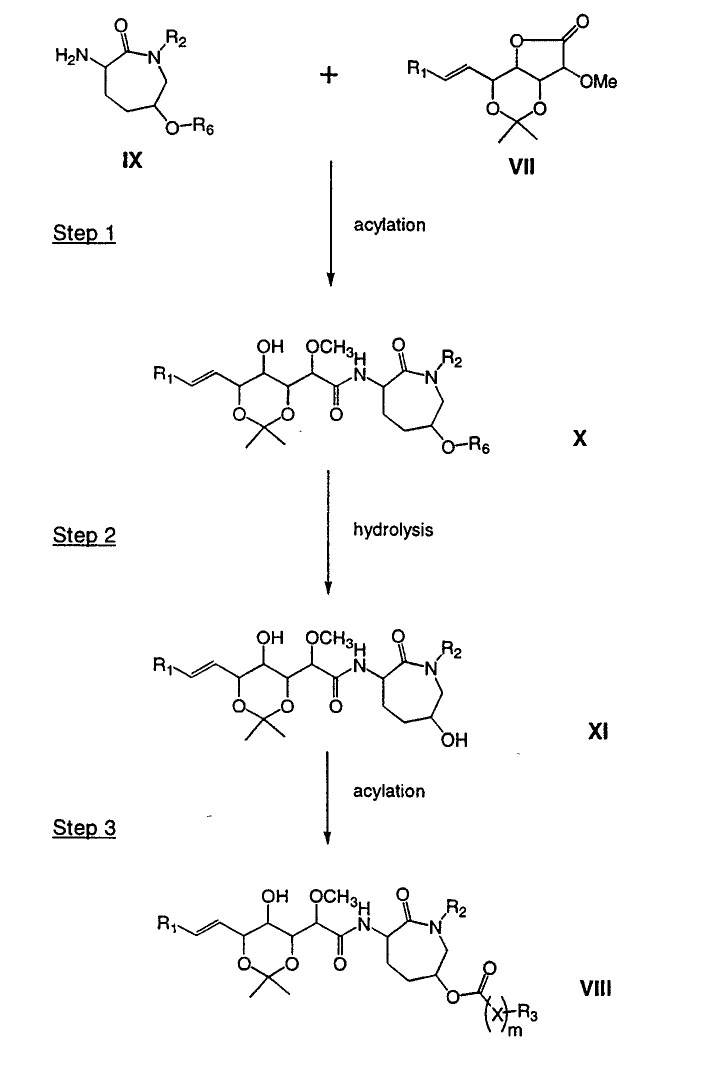 where X, m and R₃ and each R₁ and R₂ are as defined above, and R₆ is an alcohol protective group. Preferably, R₆ is a silyl group such as *tert*-butyldimethylsilyl.

As to the individual steps, Step 1 involves the acylation of an aminocaprolactam of formula IX with a lactone compound of formula VII to obtain a diamide compound of formula X. The acylation is conducted in the presence of a base, preferably an alkylamine base such as diisopropylethylamine, and a polar, organic solvent, preferably a protic polar solvent such as isopropanol, at a temperature slightly below or at the reflux temperature of the solvent employed for a period of between 4 and 48 hours.

Step 2 concerns the hydrolysis of the group R₆ common to a diamide compound of formula X to obtain a hydroxycaprolactam compound of formula XI. The hydrolysis is typically carried out in the presence of fluoride, preferably a fluoride salt such as tetrabutylammonium fluoride, and an inert organic solvent, preferably a cyclic ether such as tetrahydrofuran, at a temperature of between 0 °C and 25 °C for a period of between 5 minutes and 2 hours.

Step 3 concerns the acylation of a hydroxycaprolactam compound of formula XI by reacting it with an acid chloride of formula R₃(X)ₘCOCl where R₃, X and m are as defined above, to obtain a diamide compound of formula VIII. The acylation is conducted in the presence of a base, preferably an alkylamine base such as triethylamine, and an inert organic solvent, preferably a chlorinated alkane such as dichloromethane, at a temperature of between -78 ° C and 25 °C for a period of between 1 and 24 hours.

Alternatively, the acylation of a hydroxycaprolactam compound of formula XI in Step 3 may be carried out with a carboxylic acid of formula R₃(X)ₘCO₂H where R₃, X and m are as defined above, in the presence of a carboxylic acid coupling reagent, preferably a diimide such as 1-(3-dimethylaminopropyl)-3-ethylcarbodiimide hydrochloride, and a suitable activating agent common to diimide coupling reactions, preferably a substituted pyridine such a 4-dimethylaminopyridine, and an inert organic solvent, preferably a chlorinated alkane such as dichloromethane, at a temperature of between -78 °C and 25 °C for a period of between 1 and 24 hours.

The aminocaprolactam compounds of formula VI may be prepared as depicted below: 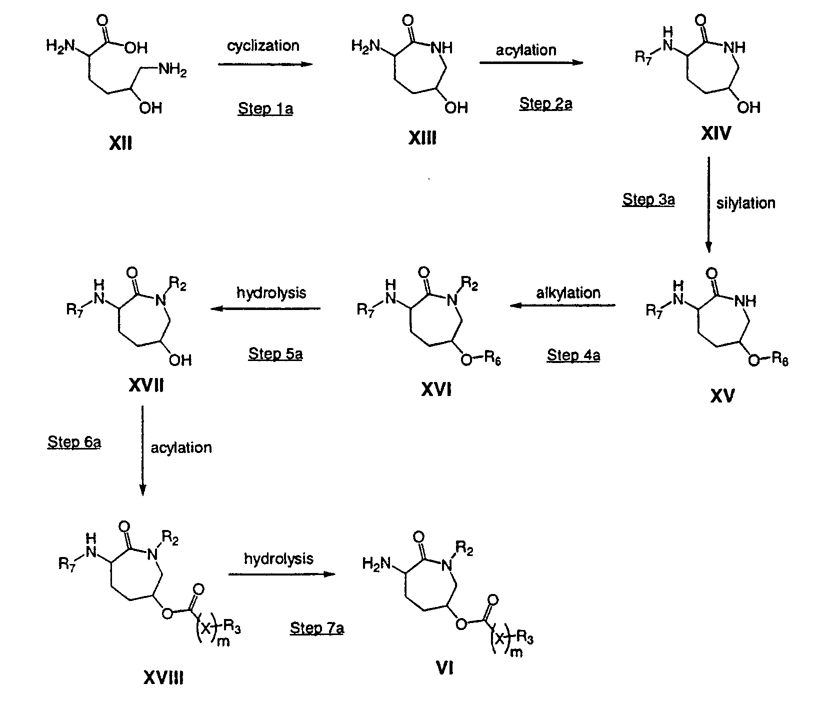 where each R₆, R₂, X, m and R₃ is as defined above, and each R₇ is a carbonyl-containing group. Preferably, R₇ is alkoxycarbonyl such as *t*-butyloxycarbonyl.

As to the individual steps, Step 1 a involves the cyclization of hydroxylysine (or any salt or hydrate preparation thereof) XII to obtain hydroxycyclolysine XIII. The cyclization is typically carried out in the presence of a coupling reagent, preferably a diimide such as 1-(3-dimethylaminopropyl)-3-ethylcarbodiimide hydrochloride, and a suitable activating agent common to diimide coupling reactions, preferably an *N*-hydroxy compound such as 1-hydroxybenztriazole hydrate, and a base, preferably an alkylamine base such as triethylamine, and a polar organic solvent, preferably an amide such as *N,N*-dimethylformamide, at a temperature of between 0 °C and 40 °C for a period of between 12 and 72 hours.

Step 2a involves the *N*-acylation of hydroxycyclolysine XIII to obtain an *N*-acylhydroxycyclolysine compound of formula XIV. The acylating agent is typically an acid chloride or an anhydride. When R₇ is *t*-butyloxycarbonyl, the acylating agent is di-*tert*-butyldicarbonate. The reaction is carried out in the presence of a base, preferably an alkylamine base such as triethylamine, and a polar organic solvent, preferably an amide such as *N,N*-dimethylformamide, at a temperature of between 0 °C and 40 °C for a period of between 1 and 24 hours.

Step 3a involves the *O*-silylation of an *N*-acylhydroxycyclolysine compound of formula XIV to obtain a silyl ether compound of formula XV. The silylating agent is typically a silyl chloride or trifluoromethanesulfonate. When R₆ is *tert*-butyldimethylsilyl, the silylating agent is *tert*-butyldimethylsilylchloride. The reaction is carried out in the presence of a base, preferably a mild base such as imidazole, and a polar organic solvent, preferably an amide such as *N,N*-dimethylformamide, at a temperature of between 0 °C and 40 °C for a period of between 1 and 24 hours.

Step 4a involves the *N*-alkylation of a silyl ether compound of formula XV with an alkyl (defined as R₂ above) halide or sulfonate to obtain an *N*-alkyl caprolactarn compound of formula XVI. The alkylation is conducted in the presence of a strong base, preferably an alkali metal amide such as sodium bis(trimethylsilyl)amide, and an inert organic solvent, preferably a cyclic ether such as tetrahydrofuran, at a temperature of between -100 °C and 25 °C for a period of between 5 minutes and 2 hours.

Step 5a concerns the hydrolysis of the group R₆ common to an N-alkyl caprolactam compound of formula XVI, to obtain a hydroxycaprolactam compound of formula XVII. The hydrolysis is typically carried out in the presence of fluoride, preferably a fluoride salt such as tetrabutylammonium fluoride, and an inert organic solvent, preferably a cyclic ether such as tetrahydrofuran, at a temperature of between 0 °C and 25 °C for a period of between 5 minutes and 2 hours.

Step 6a concerns the acylation of a hydroxycaprolactam compound of formula XVII to obtain an ester compound of formula XVIII by reacting it with an acid chloride of formula R₃(X)ₘCOCl where R₃, Xand m are as defined above, in the presence of a base, preferably an alkylamine base such as triethylamine, and an inert organic solvent, preferably a chlorinated alkane such as dichloromethane, at a temperature of between -78°C and 25 °C for a period of between 1 and 24 hours.

Alternatively, the acylation of a hydroxycaprolactam compound of formula XVII in Step 6a may be carried out with a carboxylic acid of formula R₃(X)ₘCO₂H where R₃, X and m are as defined above, in the presence of a carboxylic acid coupling reagent, preferably a diimide such as 1-(3-dimethylaminopropyl)-3-ethylcarbodiimide hydrochloride, and a suitable activating agent common to diimide coupling reactions, preferably a substituted pyridine such a 4-dimethylaminopyridine, and an inert organic solvent, preferably a chlorinated alkane such as dichloromethane, at a temperature of between -78 °C and 25 °C for a period of between 1 and 24 hours.

Step 7a concerns the hydrolysis of the group R₇ on an ester compound of formula XVIII to obtain an aminocaprolactam compound of formula VI. The hydrolysis is typically carried out in the presence of a protic acid, preferably an organic acid such as trifluoroacetic acid, hydrogen or a silyl halide, preferably a silyl iodide such as trimethylsilyl iodide, and an inert organic solvent, preferably a chlorinated alkane such as dichloromethane, at a temperature of between -100 °C and 25 °C for a period of between 1 minute and 2 hours.

The aminocaprolactam compounds of formula Via may be prepared as depicted below: 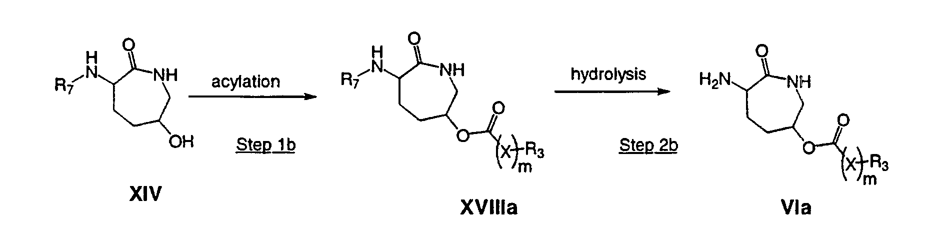 where each R₇, X, m, and R₃ is as defined above.

As to the individual steps, Step 1 b concerns the acylation of a hydroxycaprolactam compound of formula XIV to obtain an ester compound of formula XVIIIa by reacting it with an acid chloride of formula R₃(X)ₘCOCl where R₃, X and m are as defined above, in the presence of a base, preferably an alkylamine base such as triethylamine, and an inert organic solvent, preferably a chlorinated alkane such as dichloromethane, at a temperature of between -78 °C and 25 °C for a period of between 1 and 24 hours.

Alternatively, the acylation of a hydroxycaprolactam compound of formula XIV in Step 1b may be carried out with a carboxylic acid of formula R₃(X)ₘCO₂H where R₃, X and m are as defined above, in the presence of a carboxylic acid coupling reagent, preferably a diimide such as 1-(3-dimethylaminopropyl)-3-ethylcarbodiimide hydrochloride, and a suitable activating agent common to diimide coupling reactions, preferably a substituted pyridine such a 4-dimethylaminopyridine, and an inert organic solvent, preferably a chlorinated alkane such as dichloromethane, at a temperature of between -78 °C and 25 °C for a period of between 1 and 24 hours.

Step 2b concerns the hydrolysis of the group R₇ on an ester compound of formula XVIIIa to obtain an aminocaprolactam compound of formula VIa. The hydrolysis is typically carried out in the presence of an protic acid, preferably an organic acid such as trifluoroacetic acid, hydrogen or a silyl halide, preferably a silyl iodide such as trimethylsilyl iodide, and an inert organic solvent, preferably a chlorinated alkane such as dichloromethane, at a temperature of between -100 °C and 25 °C for a period of between 1 minute and 12 hours.

The aminocaprolactam compounds of formula IXa may be prepared as depicted below: 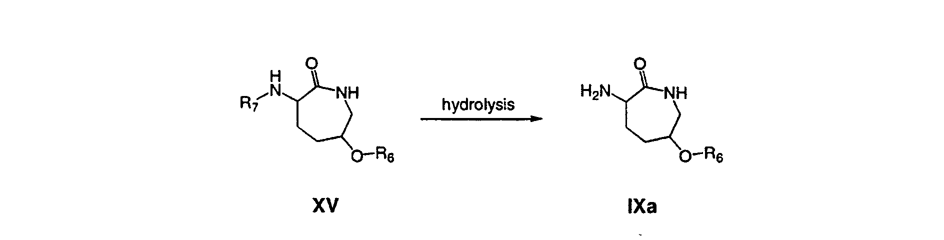 where R₇ and each R₆ are as defined above. The reaction concerns the hydrolysis of the group R₇ on an ester compound of formula XV to obtain an aminocaprolactam compound of formula IXa. The hydrolysis is typically carried out in the presence of a protic acid, preferably an organic acid such as trifluoroacetic acid, hydrogen or a silyl halide, preferably a silyl iodide such as trimethylsilyl iodide, and an inert organic solvent, preferably a chlorinated alkane such as dichloromethane, at a temperature of between -100 °C and 25 °C for a period of between 1 minute and 2 hours.

The lactone compounds of formula VII may be prepared as depicted below: 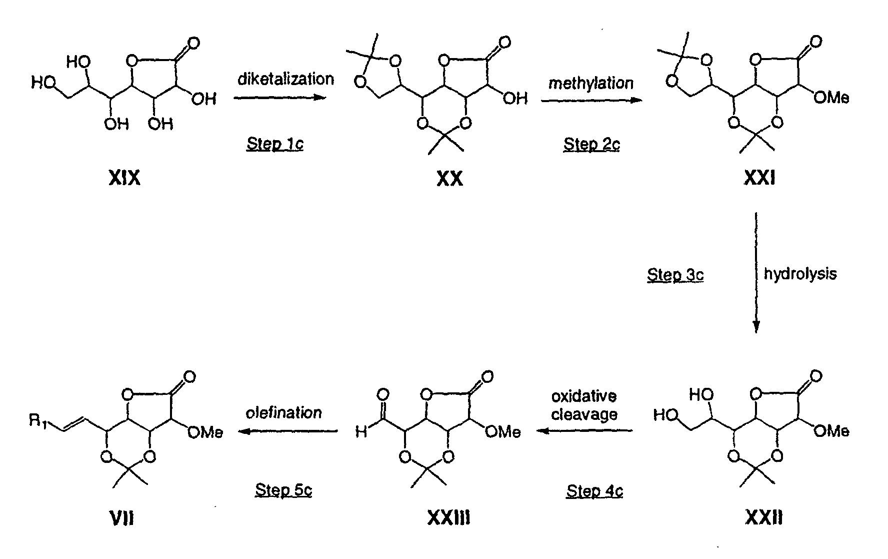 where R₁ is as defined above.

As to the individual steps, Step 1c involves the diketalization of polyhydroxylated lactone of formula XIX with acetone to obtain bis(acetonide) XX. The diketalization is conducted in acetone as solvent using a catalyst such as iodine at a temperature of between 0 °C and the reflux temperature for a period of between 2 and 48 hours.

Step 2c involves the methylation of *bis*(acetonide) XX with a methylating agent such as methyl iodide to obtain the methyl ether XXI. The methylation is conducted in the presence of water and a base, preferably a metal oxide such as silver oxide, and an inert organic solvent, preferably a chlorinated alkane such as dichloromethane, at a temperature of between 0 °C and the reflux temperature for a period of between 12 hours and 7 days.

Step 3c involves the hydrolysis of methyl ether XXI to obtain the dihydroxy compound of formula XXII. The hydrolysis is conducted in the presence of water and a protic acid, preferably a carboxylic acid such as acetic acid, at a temperature of between 5 °C and 35 C for a period of between 1 and 24 hours.

Step 4c involves the oxidative cleavage of dihydroxy compound XXII to obtain the aldehyde XXIII. The reaction is conducted in the presence of an oxidant, preferably a periodate salt such as sodium periodate, in a protic solvent, preferably an alkanol such as methanol, at a temperature of between 0 °C and 25 °C for a period of between 10 minutes and 4 hours.

Step 5c involves the olefination of aldehyde XXIII to obtain a lactone compound of formula VII. The olefination is conducted in the presence of an organometallic compound, preferably an organochromium compound such as the transient species generated from chromium(II)chloride and a diiodoalkane (defined as R₁CHI₂ where R₁ is as defined above), in the presence of a solvent mixture consisting of 1) a polar organic solvent, preferably an amide such as *N,N*-dimethylformamide, and 2) an inert organic solvent, preferably a cyclic ether such as tetrahydrofuran, at a temperature of between -80 °C and 25 °C for a period of between 5 minutes and 4 hours.

Although the product of each reaction described above may, if desired, be purified by conventional techniques such as chromatography or recrystallization (if a solid), the crude product of one reaction is advantageously employed in the following reaction without purification.

As is evident to those skilled in the art, the substituted caprolactam compounds of formula I contain asymmetric carbon atoms. It should be understood, therefore, that the individual stereoisomers are contemplated as being included within the scope of this invention.

As indicated above, certain of the compounds of formula I form pharmaceutically acceptable acid addition salts. For example, the free base of a compound of formula I can be reacted with hydrochloric acid to form the corresponding hydrochloride salt form, whereas reacting the free base of the compound of formula I with methanesulfonic acid forms the corresponding mesylate salt form. All pharmaceutically acceptable addition salt forms of the compounds of formula I are intended to be embraced by the scope of this invention.

In a further embodiment, the present invention relates to a process for preparing a caprolactam compound of formula I which comprises, in a first step, acylating an amino caprolactam compound of formula VI 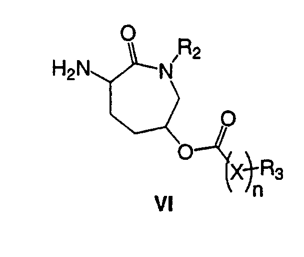 with a lactone compound of formula VII 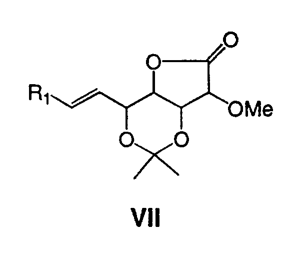 in the presence of a polar, organic solvent to obtain a diamide compound of formula VIII 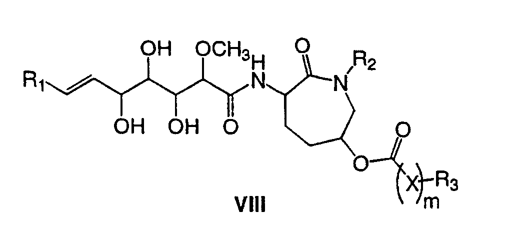 where each of R₁, R₂, X, m and R₃ are as defined above and, in a second step, hydrolyzing the diamide compound obtained in the first step by dissolving it in a mixture of solvents to obtain the desired caprolactam compound of formula I. Preferably, the acylation in the first step is conducted in isopropanol at a temperature slightly below or at the reflux temperature of the isopropanol, whereas the hydrolysis in the second step is conducted in a mixture consisting of a protic, organic acid, a protic solvent and an inert, organic solvent, more preferably a mixture consisting of trifluoroacetic acid, water and tetrahydrofuran.

As indicated above, all of the compounds of formula I, and their corresponding pharmaceutically acceptable acid addition salts, are anti-tumor agents and are, therefore, useful in inhibiting the growth of various lymphomas, sarcomas, carcinomas, myelomas, and leukemia cell lines. The anti-tumor activity of the compounds of formula I may be demonstrated employing the Anchorage Dependent Growth Monolayer Assay (ADGMA) which measures the growth inhibitory effects of test compounds on proliferation of adherent cell monolayers. This assay was adapted from the 60 cell line assay used by the National Cancer Institute (NCI) with the following modifications: 1) a single cell line representative for the important tumor type, *viz*., MDA-MB-435 breast carcinoma was utilized; 2) a tetrazolium derivative, *viz*., MTS, was utilized to determine cell density.

The ADGMA compares the number of viable cells following a 3-day exposure to a test compound relative to a number of cells present at the time the test compound was added. Cell viability is measured using a tetrazolium derivative, *viz*., 3-(4,5-dimethylthiazol-2-yl)-5-(3-carboxymethoxyphenyl)-2-(4-sulfophenyl)-2H-tetrazolium, inner salt (MTS) that is metabolically reduced in the presence of an electron coupling agent (PMS; phenazine methosulfate) by viable cells to a water-soluble formazan derivative. The absorbance at 490 nm (A₄₉₀) of the formazan derivative is proportional to the number of viable cells. The IC₅₀ for a test compound is the concentration of compound required to reduce the final cell number to 50% of the final control cell number. If cell proliferation is inhibited, the assay further defines compounds as cytostatic (cell number after 3-day compound incubation >cell number at time of compound addition) or cytotoxic (cell number after 3-day compound incubation <cell number at time of compound addition).

The MDA-MB-435 breast carcinoma line was obtained from the American Type Culture Collection (ATCC) and used between passages 4-20 following thawing. MDA-MB-435 breast carcinoma was maintained and plated in DME/F12 medium containing 10% fetal bovine serum, 15 mM HEPES (pH=7.4), penicillin 100 units/mL, and streptomycin 100 µg/mL.

Cell lines are trypsinized and counted using a Coulter counter to determine plating densities. Cells are then plated in their respective maintenance media (100 µL/well) in 96 well plates at the following densities: MDA-MB-435, 3,000 cells/well. The number of cells plates as determined in preliminary experiments, results in cell densities of 75-90% of confluency by 4 days after plating. Initial cell densities, assayed one day after plating, are roughly 0.15-0.20 absorbance units greater than the media blank. Ninety-six well plates are seeded on day 0 and the test compounds are added on day 1. A control plate is created for each cell line that receives media only in row A and cells in row B. One day following plating, test compounds are added (in a final volume of 100 µL) to the test plates. Control plates receive 10 µL MTS mixture (prepared fresh on day of addition to cell plates at a ratio of 10 µL of a 0.92 mg/mL solution of PMS to a 190 µL of a 2 mg/mL solution of MTS) and 100 µL media. A₄₉₀ of control plates is read 4h after MTS addition to determine initial cell density values for each cell line. Three days after addition of test compound, 10 µL/well of MTS mixture is added to the test plates and A₄₉₀ is read 4h later. A₄₉₀ values for wells containing cells are corrected for media absorbance, then normalized to initial density readings to determine percent net growth. IC₅₀ values are determined from graphs of percent net growth as a function of compound concentration. Percent net growth is calculated as (Cell + Drug A₄₉₀ - Initial A₄₉₀/Cell + Drug Vehicle A₄₉₀ - Initial A₄₉₀).

The following IC₅₀ values (average ± S.D.) in µM were obtained:

**Table 1**

| Compound | MDA-MB-435 |
|---|---|
| Ex. 1 | 0.068± 0.04 |
| Ex. 2 | 0.115± 0.04 |
| Ex. 3 | 0.032± 0.014 |
| Ex. 4 | 0.01± 0.01 |
| Ex. 5 | 0.465± 0.204 |
| Ex. 6 | 0.065± 0.001 |
| Ex. 7 | 0.262± 0.07 |
| Ex. 8 | 0.175± 0.007 |
| Ex. 9 | 0.335± 0.141 |
| Ex. 10 | 0.008± 0.004 |
| Ex. 11 | 0.019 ± 0.005 |
| Ex. 12 | 0.034 ± 0.046 |
| Ex. 13 | 0.30 ± 0.00 |
| Ex. 14 | 1.24 ± 0.20 |
| doxorubicin (a known antineoplastic compound) | 0.137± 0.105 |

The anti-tumor activity of the compounds of formula I may further be demonstrated employing the athymic (T cell deficient) nude mouse model which has been and remains the standard for drug discovery and development in preclinical oncology. Utilizing this model, one can measure the ability of test compounds to inhibit the growth of human tumor xenografts growing subcutaneously (s.c.) in athymic nude mice. The histologic tumor type employed was MDA-MB-435 breast carcinoma.

Briefly, 3 million cells were implanted s.c. into the right flank of athymic (nu/nu) mice, and were allowed to grow until a mass of approximately 30 mm³ was established. The test compounds are administered three times per week intravenously (i.v.) for three weeks in 5% dextrose, 10%DMSO in water. The test compounds are administered in dose-response fashion in order to evaluate and document the full potential range of activity (efficacy and toxicity) for a given compound. Positive controls are carried out with doxorubicin administered 3 times per week i.v.

Toxicity was monitored by recording average group body weights twice weekly, and by daily observation of general health. Efficacy was monitored by taking measurements of tumor length, width, and depth weekly using digital calipers coupled to automated data collectors. Mean tumor volume (MTV) at initiation of therapy was subtracted from final MTV in order to express the actual tumor growth during treatment (Δ MTV). Anti-tumor activity was expressed as %T/C (Δ MTV of treated group ö Δ MTV of control group X100). Statistical significance was evaluated using a one-tailed Student's t-test (p<0.05).

The following results were obtained for compounds Ex.1 - Ex.10 tested against MDA-MB-435 tumor xenografts 3X/week for 3 weeks:

**Table 2**

| Compound | Dose (µmol/kg) | Δ MTV (mm³) | %T/C | Dead/Total |
|---|---|---|---|---|
| Ex.1 | 10 | 107 | 61* | 0/8 |
| Ex.1 | 33 | 43 | 24* | 0/8 |
| Ex.1 | 100 | -11 | -6* | 0/8 |
| Ex.2 | 10 | 94 | 67 | 0/8 |
| Ex.2 | 33 | 19 | 13* | 0/8 |
| Ex.3 | 10 | 69 | 39* | 0/8 |
| Ex.3 | 33 | 49 | 28* | 0/8 |
| Ex.4 | 10 | 93 | 53* | 0/8 |
| Ex.4 | 33 | 21 | 12* | 0/8 |
| Ex.4 | 100 | -23 | -13* | 0/8 |
| Ex.5 | 10 | 130 | 92 | 0/8 |
| Ex.5 | 33 | 62 | 44* | 0/8 |
| Ex.6 | 10 | 66 | 47* | 0/8 |
| Ex.6 | 33 | 48 | 34* | 0/8 |
| Ex.7 | 10 | 101 | 72 | 0/8 |
| Ex.7 | 33 | 61 | 43* | 0/8 |
| Ex.8 | 10 | 87 | 62 | 0/8 |
| Ex.8 | 33 | 94 | 67 | 0/8 |
| Ex.9 | 10 | 107 | 76 | 0/8 |
| Ex.9 | 33 | 68 | 48* | 0/8 |
| Ex.10 | 10 | 115 | 64* | 0/8 |
| Ex. 10 | 33 | 4 | 2* | 0/8 |
| Ex. 11 | 10 | 135 | 76 | 0/8 |
| Ex. 11 | 33 | 76 | 43* | 0/8 |
| Ex. 12 | 10 | 78 | 52 | 0/8 |
| Ex. 12 | 33 | 11 | 7* | 0/8 |
| doxorubicin | 2 mg/kg | 74 | 42* | 0/8 |

| | | | | |
|---|---|---|---|---|
| * %T/C values were statistically significant (p=<0.05; Student's t-test). | | | | |

The precise dosage of the compounds of formula I to be employed for inhibiting tumors depends upon several factors including the host, the nature and the severity of the condition being treated, the mode of administration and the particular compound employed. However, in general, satisfactory inhibition of tumors is achieved when a compound of formula I is administered parenterally, e.g., intraperitoneally, intravenously, intramuscularly, subcutaneously, intratumorally, or rectally, or enterally, e.g., orally, preferably intravenously or orally, more preferably intravenously at a daily dosage of 1-300 mg/kg body weight or, for most larger primates, a daily dosage of 50-5000, preferably 500-3000 mg. A preferred intravenous daily dosage is 1-75 mg/kg body weight or, for most larger primates, a daily dosage of 50-1500 mg. A typical intravenous dosage is 20 mg/kg, three to five times a week.

Usually, a small dose is administered initially and the dosage is gradually increased until the optimal dosage for the host under treatment is determined. The upper limit of dosage is that imposed by side effects and can be determined by trial for the host being treated.

The compounds of formula I may be combined with one or more pharmaceutically acceptable carriers and, optionally, one or more other conventional pharmaceutical adjuvants and administered enterally, e.g. orally, in the form of tablets, capsules, caplets, etc. or parenterally, e.g., intraperitoneally or intravenously, in the form of sterile injectable solutions or suspensions. The enteral and parenteral compositions may be prepared by conventional means.

The infusion solutions according to the present invention are preferably sterile. This may be readily accomplished, e.g. by filtration through sterile filtration membranes. Aseptic formation of any composition in liquid form, the aseptic filling of vials and/or combining a pharmaceutical composition of the present invention with a suitable diluent under aseptic conditions are well known to the skilled addressee.

The compounds of formula I may be formulated into enteral and parenteral pharmaceutical compositions containing an amount of the active substance that is effective for inhibiting tumors, such compositions in unit dosage form and such compositions comprising a pharmaceutically acceptable carrier.

The following examples show representative compounds encompassed by this invention and their synthesis.

### EXAMPLE 1: (2R, 3R, 4S, 5R, 6E)-3,4,5-trihydroxy-2-methoxy-8,8-dimethyl-N-[(3S, 6R)-hexahydro-2-oxo-6-(cyclohexylcarbonyl)oxy-2H-azepin-3-yl]non-6-enamide

### a) Preparation of 3,5:6,7-bis-O-(1-methylethylidene)-α-D-glucoheptonic γ- lactone.

α-D-Glucoheptonic γ-lactone (500 g, 2.4 mol) is added into 9 L of acetone in a 5 gal plastic drum. The mixture is agitated mechanically until most of the solid dissolved (15-20 min). Iodine (60g, 0.236 mol) is added portionwise into the lactone soln over 5-10 min. The resulting mixture is stirred overnight. A saturated soln of Na₂S₂O₃ (1.3 L) is added to the iodine soln to quench the reaction. The resulting soln is concd to about half of its original volume *in vacuo*, and brine soln (5 L) is added. The resulting mixture is extracted with 3 x 1.2 L EtOAc. All organic layers are combined and evaporated to dryness. The solid is slurried with a mixture of ether and hexane (3:7), and filtered. The filter cake is washed with Et₂O (50 mL) and air dried, giving 599 g of the desired compound as a white powder (86.5%): ¹H NMR (CDCl₃) δ 4.62 (m, 1H), 4.50 (m, 1H), 4.35 (m, 2H), 4.07 (m, 1H), 3.93 (m, 1H), 3.82 (dd, 1H), 3.08 (d, 1H), 1.51 (s, 3H), 1.44 (s, 3H), 1.39 (s, 3H), 1.35 (s, 3H); ¹³C NMR (CDCl₃) δ 174.4, 109.4, 98.6, 72.8, 71.4, 69.3, 68.4, 67.8, 66.7, 28.6, 26.7, 24.6, 19.3.

### b) Preparation of 2-O-methyl-3,5:6,7-bis-O-(1-methylethylidene)-α-D-glucoheptonic γ-lactone.

3,5:6,7-bis-*O*-(1-methylethylidene)-α-D-glucoheptonic γ- lactone (719 g, 2.49 mol) is added into 4.5 L of CH₂Cl₂ in a 5 gal plastic drum. The mixture is stirred under N₂. lodomethane (2500 g, 17.6 mol) is added immediately followed by addition of silver(I)oxide (1750 g, 7.58 mol). Water (30 mL) is added to the reaction mixture. Ice bath is used to maintain the reaction temp at 15-30 °C. The reaction is stirred in the absence of light for 18 h. After diluting the reaction mixture with 1.5 L of CH₂Cl₂, the solid is filtered and washed with an additional 2.2 L of CH₂Cl₂. The undesired solid is discarded and the filtrate is evaporated to dryness. The residue is slurried in Et₂O (1.5 L), filtered, and dried to give 618 g product (82 %): ¹H NMR (CDCl₃) δ 4.75 (m, 1H), 4.33 (m, 1H), 4.29 (m, 1H), 4.15 (m, 1H), 4.07 (m, 1H), 3.96 (dd, 1H), 3.83 (dd, 1H), 3.65 (s, 3H), 1.57 (s, 3H), 1.42 (s, 6H), 1.35 (s, 3H); ¹³C NMR (CDCl₃) δ 172.5, 109.6, 98.5, 79.0, 73.1, 69.5, 68.6, 67.5, 66.9, 59.1, 28.9, 26.9, 24.9, 19.4.

### c) Preparation of 2-O-Methyl-3,5-O-(1-methylethylidene)-α-D-glucoheptonic γ- lactone.

2-*O*-methyl-3,5:6,7-bis-*O*-(1-methylethylidene)-α-D-glucoheptonic γ- lactone (618 g, 2.05 mol) is dissolved in 8 L of a mixture of acetic acid and water (1:1) over 30 min. The soln is stirred at ambient temp overnight. The soln is evaporated to dryness *in vacuo*. The solid is slurried in 3-5 L of hot acetone and filtered. After oven drying at 20-30 °C, 363 g of the desired compound is obtained (67.6 %). ¹H NMR(CDCL₃): δ 4.92 (d, 1H), 4.80 (m, 1H), 4.47 (d, 1H), 4.42 (t, 1H), 4.39 (m, 1H), 3.95 (dd, 1H), 3.75 (m, 2H), 3.4 (s, 3H), 2.5 (m, 1H), 1.42 (s, 3H), 1.22 (s, 3H).

### d) Preparation of 2,4-O-(1-methylethylidene)-5-O-methyl-L-glucuronic γ- lactone.

2-*O*-Methyl-3,5-*O*-(1-methylethylidene)-α-D-glucoheptonic γ- lactone (200 g, 0.76 mol) is dissolved into a 1:1 mixture of methanol and water (3.6 L). The stirred mixture is cooled in an ice water bath to about 8 °C. Solid NaIO₄ (213 g, 0.98 mol) is added portionwise. Reaction is complete within 40 min as indicated by TLC (silica gel, 5%methanol, 15% EtOAc in CH₂Cl₂). Solid NaCl is added into the reaction mixture to saturate the methanolic soln. The solid is filtered and washed with 2 L CH₂Cl₂. The filtrate is extracted with 7x500 mL CH₂Cl₂. Combined organic layers are dried over Na₂SO₄, filtered and concd to a syrup, which formed a precipitate upon addition of hexane. The solid is filtered and rinsed with Et₂O. A portion of the crude product (50 g) is dissolved in 3 L CHCl₃ and heated to reflux. After rotary evaporation of 2.1 L of CHCl₃ at atmospheric pressure (methanol is driven out of the system by coevaporation with CHCl₃) the residue is evaporated to dryness. 44 g of the desired product is obtained as a solid after drying *in vacuo* overnight. ¹H NMR (CDCl3): δ 9.60 (s, 1H), 4.78 (m, 1H), 4.42 (s, 2H), 4.15 (dd, 1H), 3.65 (s, 3H), 1.58 (s, 3H), 1.55 (s, 3H); ¹³C NMR (CDCl₃) δ 198.8, 171.9, 99.0, 78.4, 74.4, 72.9, 68.4, 67.4, 59.2, 28.7, 19.0,

### e) Preparation of (6E)-6,7,8,9-tetradeoxy-8,8-dimethyl-2-O-methyl-3,5-O-(1-methylethylidene)-gulo-non-6-enonic acid lactone.

Into a 2 L round bottom flask, is added CrCl₂ (50 g, 41 mmol ), anhydrous THF (750 mL), and DMF (32 mL). The mixture is stirred under N₂ for 1 h. A soln of 2,4-*O*-(1-methylethylidene)-5-*O*-methyl-L-glucuronic γ- lactone (12 g, 50 mmol), 1,1-diiodo-2,2-dimethylpropane (15 mL), and 500 mL of anhydrous THF is added slowly into the reaction mixture. After the addition, the reaction mixture is stirred at ambient temp for 1.5 h. The reaction is quenched with satd. aq. NH₄Cl. The residue is partitioned with EtOAc/water and chromatographed (5% EtOAc - CH₂Cl₂) to give 9 g (63%) of the desired compound as a white crystalline solid: ¹HNMR (CDCl₃) δ 5.82 (d, 1H), 5.58 (q, 1H), 4.71 (m, 1H), 4.46 (m, 1H), 4.10 (dd, 1H), 4.0 (m, 1H), 3.66 (s, 3H), 1.58 (s, 3H), 1.53 (s, 3H), 1.07 (s, 9H); ¹³C NMR (CDCl₃) δ 172.5, 147.0, 120.2, 98.7, 79.1, 71.9, 70.3, 67.6, 59.2, 33.2, 29.3, 19.3.

### f) Preparation of (3S, 6R)-3-(tert-butoxycarbonyl)aminohexahydro-6-hydroxy-2H-azepin-2-one

In a 1 I flask (5*R*)-5-hydroxy-L-lysine (10 g, 0.040 mol), 1-hydroxybenzotriazole hydrate (8.2 g, 0.060 mol) and 1-[3-(dimethylamino)propyl]-3-ethylcarbodiimide-HCl (11.6 g, 0.060 mol) are added to 500 ml DMF with stirring. After 0.5 h triethylamine (16.8 ml, 0.120 mol) is added. The reaction is stirred at rt for 48 h. Di-*tert*-butyl dicarbonate (17.6 g, 0.080 mol) and triethylamine (16.8 ml, 0.120 mol) are added. Stirring is continued for 16 h. The reaction mixture is filtered to remove triethylamine-HCl and the solvent is removed by rotary evaporation under high vacuum to give a thick oil. The oil is dissolved in 150 ml CH₂Cl₂ and applied to a silica gel column (150 g, 40 x 250 mm). The column is eluted with 3% methanol in CH₂Cl₂ to give the crude product as a solid. The crude solid is dissolved in 120 ml hot CH₂Cl₂ and cooled to -20 °C for 1 h. The resulting solid is filtered and washed with 50 ml CH₂Cl₂. The combined filtrates are evaporated to dryness. CH₂Cl₂ (40 ml) is added to this residue and the resulting slurry is stirred for 0.5 h at rt. The slurry is filtered and the solid washed with 25 ml CH₂Cl₂. The solids are combined to give 5.57 g of (3*S*, 6*R*)-3-(*tert*-butoxycarbonyl)aminohexahydro-6-hydroxy-2*H*-azepin-2-one. 300 MHz ¹H NMR (DMSO) δ 7.42 (1H, t, J = 5.1 Hz), 6.38 (1H, d, J = 6.6 Hz), 4.60 (1H, d, J = 4.2 Hz), 4.07 (1H, m), 3.74 (1H, m), 3.32 (1H, m), 3.03 (1H, m), 1.8-1.5 (4H, m), 1.39 (9H, s).

### g) Preparation of (3S, 6R)-3-(tert-butoxycarbonyl)aminohexahydro-6-(cyclohexanecarbonyl)oxy-2H-azepin-2-one.

Triethylamine ( 8.4 mL, 60 mmol) is added to a solution of cyclohexanecarbonyl chloride (6.3g, 43.0 mmol), (3*S*, 6*R*)-3-(*tert*-butoxycarbonyl)aminohexahydro-6-hydroxy-2*H*-azepin-2-one (7.0 g, 28.7mmol) and 100 mL of CH₂Cl₂ at 5°C. The reaction mixture is stirred at room temp. overnight. The reaction mixture is then partitioned with water, and the organic layer is dried (Na₂SO₄), and concentrated with rotary evaporation. The resulting residue is chromatographed (5% EtOAc - CH₂Cl₂) to give 10.1 (99.5% of (3*S*, 6*R*)-3-(*tert*-butoxycarbonyl)aminohexahydro-6-(cyclohexanecarbonyl)oxy-2*H*-azepin-2-one as a white solid: ¹H NMR (CDCl₃): δ 5.89 (d, J=5.27Hz, 1H), 5.65 (t, J=4.90 Hz, 1H), 4.89 (s, 1H), 4.30 (q, J=4.14Hz, 1H), 3.49 (m, 2H), 2.31 (tt, J=10.92Hz and 3.39Hz, 1H), 2.13 (d, J=14.32Hz, 1H), 1.98 (d, J=13.56Hz, 2H), 1.88 (d, J=14.31Hz, 2H), 1.75 (d, J=11.30Hz, 2H), 1.66 (s, 2H), 1.45 (s, 9H), 1.30 (m, 5H).

### h) Preparation of (3S, 6R)-3-aminohexahydro-6-(cyclohexanecarbonyl)oxy-2H-azepin-2-one.

To a solution of (3*S*, 6*R*)-3-(*tert*-butoxycarbonyl)aminohexahydro-6-(cyclohexanecarbonyl)oxy-2*H*-azepin-2-one (10g, 28.2 mmol) in 40 mL of CH₂Cl₂ is added TFA (25mL) at room temp., and the reaction solution is stirred at room temp. for 1 hr, then concd via rotary evaporation (bath temp<20 °C). The residue is diluted with CH₂Cl₂ (100 mL), and washed with NH₄OH (10 mL) and then water (2x20mL) and dried (Na₂SO₄). The reaction mixture is adsorbed on silica and chromatographed ( 5% methanol-CH₂Cl₂) to give 6.0 (85.0%) of (3*S*, 6*R*)-3-aminohexahydro-6-(cyclohexanecarbonyl)oxy-2*H*-azepin-2-one as a white solid: ¹H NMR (CDCl₃): δ 6.91 (s, 1H), 4.91 (s, 1H), 4.39 (s, 2H), 3.87 (d, J=9.80Hz, 1H), 3.48 (t, J=6.02Hz, 1H), 3.43 (dd, J=15.45Hz and 4.90Hz, 1H), 2.30 (tt, J=10.92Hz and 3.39Hz, 1H), 2.13 (m, 1H), 1.91 (m, 4H), 1.73 (m, 2H), 1.65 (m, 1H), 1.40 (q, J=11.68Hz, 4H), 1.24 (m, 2H).

### i) Preparation of (2R, 3R, 4S, 5R, 6E)-3,5-(methylethylidene)-3,4,5-trihydroxy-2-methoxy-8,8-dimethyl-N-[(3S, 6R)- hexahydro-2-oxo-6-(cyclohexylcarbonyl)oxy-2H-azepin-3-yl]non-6-enamide.

A soln consisting of (6*E*)-6,7,8,9-tetradeoxy-8,8-dimethyl-2-*O*-methyl-3,5-*O*-(1-methylethylidene)-gulo-non-6-enonic acid lactone (1.0 g, 3.5 mmol), (3*S*, 6*R*)-3-aminohexahydro-6-cyclohexanecarboxy-2*H*-azepin-2-one (2.5 g, 9.8 mmol), and *i*-PrOH (4 mL) is stirred at reflux for 24 h. The reaction mixture is adsorbed on silica and chromatographed (2% methanol - CH₂Cl₂) to give 1.85 g (97%) of the desired compound as a white solid: ¹H NMR (CDCl₃) δ 7.58 (d, J=6.341 Hz, 1H), 5.80 (t, J=7.68Hz, 1H), 5.78 (d, J=15.83Hz, 1H), 5.53 (dd, J=15.83Hz and 6.78Hz 1H), 4.92 (sd, J=3.39Hz, 1H), 4.60 (dd, J=0.42Hz and 7.4Hz, 1H), 4.28 (d, J=6.79Hz, 1H), 4.07 (dd, J=7.54Hz and 1.13Hz, 1H), 3.90 (d, J=7.15Hz, 1H), 3.52 (dd, J=12.05Hz and 7.91 Hz, 2H), 3.48 (s, 3H), 2.82 (d, J=9.04Hz, 1H), 2.32 (m, 1H), 2.12(m,1H), 2.00 (m, 2H), 1.89 (d, J=13.06Hz, 2H), 1.75 (m, 4H), 1.66 (m, 1H), 1.46 (d, J=4.90Hz, 6H),1.38 (m, 2H), 1.26 (m, 3H), 1.03 (s. 9H).

### j) Preparation of the title compound.

(2*R*, 3*R*, 4*S*, 5*R*, 6*E*)-3,5-(methylethylidene)-3,4,5-trihydroxy-2-methoxy-8,8-dimethyl-*N*-[(3*S*, 6*R*)-hexahydro-2-oxo-6-(cyclohexylcarbonyl)oxy-2*H*-azepin-3-yl]non-6-enamide (3.8 g, 7.1 mmol) is added in one portion to a stirred soln of TFA (10 mL), THF (10 mL), and water (5 mL) at 0 °C. The reaction is stirred at this temp for 30 min, concd via rotary evaporation (bath temp <20 °C), mixed with saturated NH₄HCO₃ (5 mL), and stirred for 15 min. The mixture is concd *in vacuo* and chromatographed (2% methanol - CH₂Cl₂) to give a white solid with H₂O solubility of 3.7 mg/mL. This material is further purified using preparative hplc (reverse phase eluted with 90% CH₃CN - water) to give 2.9 g (82.4%) of the title compound as a white solid, mp 79 - 80 °C; ¹H NMR (CDCl₃) δ 8.00 (d, J=6.30Hz, 1H), 5.98 (t, J=5.52Hz, 1H), 5.83 (d, J=15.77Hz, 1H), 5.42 (dd, J=15.76Hz and 7.25Hz 1H), 4.93 (m, 1H), 5.56 (m, 1H), 4.22 (m, 2H), 3.82 (m, 2H), 3.81 (t, J=5.99Hz, 1H), 3.55 (s,3H), 3.49 (dd, J=15.77Hz and 5.36Hz, 1H), 3.30 (d, J=7.25Hz, 1H), 3.10 (s, 1H), 2.31 (m, 1H), 2.16 (d, J=11.19Hz, 1H), 2.00 (m,2H), 1.88 (m, 3H), 1.76 (s, 2H), 1.65 (d, J=0.87Hz, 1H), 1.42 (m, 2H), 1.25 (m, 4H), 1.02 (s. 9H); ¹³C NMR (CDCl₃) δ 175.19, 174.11, 172.12, 145.74, 123.20, 81.10, 74.50, 72.75, 72.45, 66.74, 59.93, 51.66, 43.31, 43.22, 33.03, 31.96, 29.43, 29.07, 29.00, 25.70, 25.65, 25.39, 25.36.

### EXAMPLE 2: (2R, 3R, 4S, 5R, 6E)-3,4,5-trihydroxy-2-methoxy-8,8-dimethyl-N-[(3S, 6R)-hexahydro-2-oxo-6-(cyclopentylcarbonyl)oxy-2H-azepin-3-yl]non-6-enamide.

Following essentially the procedure of Example 1g) and using in place of cyclohexanecarbonyl chloride, an approximately equivalent amount of cyclopentanecarbonyl chloride, (3*S*, 6*R*)-3-(*tert*-butoxycarbonyl)aminohexahydro-6-(cyclopentanecarbonyl)oxy-2*H*-azepin-2-one is obtained. Employing the compound above in place of compound 1g), and following essentially the procedure of Example 1h), 1i) and the last step of Example 1, the title compound is obtained. H₂O solubility = 18 mg/mL; mp 75 - 76 °C; ¹H NMR (DMSO): δ 7.81 (d, J=6.47Hz, 1H), 7.76 (t, J=6.07Hz, 1H), 5.64 (d, J=15.77Hz,1H), 5.34 (dd, J=15.76Hz and 2.84Hz, 1H), 4.80 (s, 1H), 4.57 (d, J=4.73Hz,1H), 4.48 (d, J=6.94Hz, 1H), 4.45 (m, 1H), 4.36 (d, J=5.83Hz, 1H), 3.98 (m, 1H), 3.71 (d, J=6.94Hz 1H), 3.57 (td, J=6.78Hz and 2.68Hz, 1H), 3.52 (dd, J=15.61Hz and 4.57Hz, 1H), 3.34 (td, 6.15Hz and 2.84Hz, 1H), 3.32 (s, 3H), 3.23 (m, 1H), 2.72 (m, 1H), 2.50 (m, 1H), 1.93 (m, 2H), 1.80 (m, 2H), 1.73 (m, 3H), 1.61 (m, 2H), 1.53 (m, 2H), 0.98 (s, 9H); ¹³C NMR (DMSO): δ 174.75, 173.56, 169.73, 141.69, 125.32, 81.60, 72.82, 72.54, 70.80, 67.20, 57.31,50.95, 43.21, 32.44, 31.31, 29.45, 29.38, 25.30, 25.23.

### EXAMPLE 3: (2R, 3R, 4S, 5R, 6E)-3,4,5-trihydroxy-2-methoxy-8,8-dimethy)-N-[(3S, 6R)-hexahydro-2-oxo-6-(cycloheptylcarbonyl)oxy-2H-azepin-3-yl]non-6-enamide.

Following essentially the procedure of Example 1g) and using in place of cyclohexanecarbonyl chloride, an approximately equivalent amount of a mixture consisting of: cycloheptanecarboxylic acid , 1-(3-dimethylaminopropyl)-3-ethylcarbodiimide hydrochloride and 4-dimethylaminopyridine; (3*S*, 6*R*)-3-(*tert*-butoxycarbonyl)aminohexahydro-6-(cycloheptanecarbonyl)oxy-2*H*-azepin-2-one is obtained. Employing the compound above in place of compound 1g), and following essentially the procedure of Example 1h), 1i) and the last step of Example 1, the title compound is obtained: mp 84 - 86 °C; ¹H NMR (CDCl₃): δ 8.00 (d, J=6.30Hz, 1H), 5.93 (t, J=5.52Hz, 1H), 5.83 (d, J=15.77Hz, 1H), 5.42 ( dd, J=15.7Hz and 7.26Hz, 1H), 4.93 (m, 1H), 4.55 (dd, J=9.46Hz and 6.31 Hz, 1H), 4.23 (m, 2H), 3.82 (m,2H), 3.61 (t, J=6.14Hz, 1H), 3.57 (m, 1H), 3.55 (s, 3H), 3.49 (dd, J=15.60Hz and 5.20Hz, 1H), 3.29 (d, J=7.25Hz,1H), 3.10 (s, 1H), 2.50 (m, 1H), 2.17 (m, 1H), 1.95 (m, 4H), 1.70 (m, 1H), 1.65 (m, 2H), 1.55 (m, 4H), 1.47 (m, 4H), 1.02 (s, 9H); ¹³C NMR (CDCl₃): δ 176.17, 174.07, 172.14, 145.75, 123.20, 81.08, 74.51, 72.76, 72.44, 66.79, 59.95, 51.67, 45.07, 43.36, 33.03, 31.95, 30.91, 30.84, 29.43, 28.24, 28.21, 26.26, 25.73.

### EXAMPLE 4: (2R, 3R, 4S, 5R, 6E)-3,4,5-trihydroxy-2-methoxy-8,8-dimethyl-N-[(3S, 6R)-hexahydro-2-oxo-6-(1-oxo-3-phenylpropoxy)-2H-azepin-3-yl]non-6-enamide.

Following essentially the procedure of Example 1g) and using in place of cyclohexanecarbonyl chloride, an approximately equivalent amount of 3-phenylpropionyl chloride, (3*S*, 6*R*)-3-(*tert*-butoxycarbonyl)aminohexahydro-6-(1-oxo-3-phenylpropoxy)-2*H*-azepin-2-one is obtained. Employing the compound above in place of compound 1 g), and following essentially the procedure of Example 1 h), 1 i) and the last step of Example 1, the title compound is obtained: H₂O solubility = 0.8 mg/mL; mp 75 - 77 °C; ¹H NMR (CDCl₃) δ 7.96 (d, J=6.15Hz, 1H), 7.31 (t, J=7.25Hz,2H), 7.23 (t, J=7.57Hz, 1H), 7.20 (d, J=7.41 Hz, 2H), 5.82 (d, J=15.76Hz,1H), 5.62 (s, 1H), 5.41 (dd, J=15.76Hz and 7.09Hz, 1H), 4.92 (s, 1H), 4.49 (dd, J=9.46Hz and 6.31 Hz, 1H), 4.22 (m, 2H), 3.80 (m, 2H), 3.60 (s, 1H), 3.52 (s, 3H), 3.37 (m,3H), 3.17 (s, 1H), 2.95 (t, J=7.57Hz, 2H), 2.67 (t, J=7.72Hz, 2H), 2.06 (d, J= 11.98Hz, 1H), 1.96 (t, J=12.77Hz, 1H), 1.88 (s, 1H), 1.70 (m, 1H), 1.02 (s, 9H); ¹³C NMR (CDCl₃): δ 173.96, 172.05, 172.00 145.68, 140.08, 128.61, 128.42, 126.52, 123.21, 81.18, 74.46, 72.69, 72.50, 67.14, 59.83, 51.49, 43.22, 35.78, 33.02, 31.79, 31.07, 29.43, 25.58.

### EXAMPLE 5: (2R, 3R, 4S, 5R, 6E)-3,4,5-trihydroxy-2-methoxy-8,8-dimethyl-N-[(3S, 6R)-hexahydro-2-oxo-6-(1-oxo-3-[3-pyridyl]propoxy)-2H-azepin-3-yl]non-6-enamide.

Following essentially the procedure of Example 1 g) and using in place of cyclohexanecarbonyl chloride, an approximately equivalent amount of a mixture consisting of: 3-(pyridyl)propionic acid, 1-(3-dimethylaminopropyl)-3-ethylcarbodiimide hydrochloride and 4-dimethylaminopyridine; (3*S*, 6*R*)-3-(*tert*-butoxycarbonyl)aminohexahydro-6-(1-oxo-3-[3-pyridyl]propoxy)-2*H*-azepin-2-one is obtained. Employing the compound above in place of compound 1g), and following essentially the procedure of Example 1h), 1i) and the last step of Example 1, the title compound is obtained. ¹H NMR (CDCl₃) δ 8.46 (s, 2H), 7.97 (d, J=6.2 Hz, 1H), 7.53 (m, 1H), 7.23 (m, 1H), 5.88 (t, J=6.0 Hz, 1H), 5.81 (d, J=15.6 Hz, 1H), 5.4 (dd, J=15.6 and 7.3 Hz, 1H), 4.93 (m, 1H), 4.27 (s, 1H), 4.23-4.19 (m, 1H), 3.8 (m, 2H), 3.59 (d, J=5.2 Hz, 1H), 3.52 (s, 3H), 3.48 (m, 2H), 3.28 (s, 1H), 3.19 (s, 1H), 2.95 (t, J=7.3 Hz, 2H), 2.67 (t, J=7.3, 2H), 2.1-1.67 (m, 6H), 1.0 (s, 9H); ¹³C NMR (CDCl₃) δ 174.0, 172.0, 171.5, 149.7, 148.0, 145.7, 136.0, 135.5, 123.6, 123.2, 81.2, 74.5, 72.7, 72.5, 67.6, 59.8, 51.6, 43.3, 35.2, 33.0, 31.8, 29.4, 28.0, 25.5. HPLC: C-18 Novapac, 4.6X250 mm, 1.5 mL/min; solvent system: 25%MeCN / 75%H₂O [isocratic]; retention time = 13.7 min.

### EXAMPLE 6: (2R, 3R, 4S, 5R, 6E)-3,4,5-trihydroxy-2-methoxy-8,8-dimethyl-N-[(3S, 6R)-hexahydro-2-oxo-6-(cyclohexylmethylcarbonyl)oxy-2H-azepin-3-yl]non-6-enamide.

Following essentially the procedure of Example 1 g) and using in place of cyclohexanecarbonyl chloride, an approximately equivalent amount of a mixture consisting of: cyclohexylacetic acid , 1-(3-dimethylaminopropyl)-3-ethylcarbodiimide hydrochloride and 4-dimethylaminopyridine; (3*S*, 6*R*)-3-(*tert*-butoxycarbonyl)aminohexahydro-6-(cyclohexylmethylcarbonyl)oxy-2*H*-azepin-2-one is obtained. Employing the compound above in place of compound 1g), and following essentially the procedure of Example 1h), 1i) and the last step of Example 1, the title compound is obtained. ¹H NMR (CDCl₃) δ 7.98 (d, J=6.2, 1H), 5.83-5.7 (m, 1H), 5.80 (d, J=15.6 Hz, 1H), 4.94 (m, 1H), 4.52 (m, 1H), 4.2 (m, 2H), 3.78 (m, 2H), 3.6-3.48 (m, 3H), 3.52 (s, 3H), 3.23 (d, J=7.3 Hz, 1H), 3.06 (m, 1H), 2.80 (d, J=7 Hz, 2H), 2.13-1.63 (m, 12H), 1.30-0.85 (m, 5H), 1.0 (s, 9H); ¹³C NMR (CDCl₃) δ 174.0, 172.3, 172.2, 145.7, 123.3, 81.1, 74.5, 72.8, 72.5, 67.0, 60.0, 51.7, 43.5, 42.1, 35.0, 33.0, 32.0, 29.5, 26.1, 26.0, 25.7. HPLC: C-18 Novapac, 4.6X250 mm, 1.5 mL/min; solvent system: 40%MeCN / 60%H₂O [isocratic]; retention time = 11.5 min.

### EXAMPLE 7: (2R, 3R, 4S, 5R, 6E)-3,4,5-trihydroxy-2-methoxy-8,8-dimethyl-N-[(3S, 6R)-hexahydro-2-oxo-6-(1-oxo-2-phenylethoxy)-2H-azepin-3-yl]non-6-enamide.

Following essentially the procedure of Example 1 g) and using in place of cyclohexanecarbonyl chloride, an approximately equivalent amount of a mixture consisting of: phenylacetic acid, 1-(3-dimethylaminopropyl)-3-ethylcarbodiimide hydrochloride and 4-dimethylaminopyridine; (3*S*, 6*R*)-3-(*tert*-butoxycarbonyl)aminohexahydro-6-(1-oxo-2-phenylethoxy)-2*H*-azepin-2-one is obtained. Employing the compound above in place of compound 1g), and following essentially the procedure of Example 1h), 1i) and the last step of Example 1, the title compound is obtained. ¹H NMR (CDCl₃) δ 7.96 (d, J=6.2, 1H), 7.28 (m, 5H), 5.80 (d, J=15.8, 1H), 5.68 (t, J=6.4Hz, 1H), 5.39 (dd, J=15.8 and 7.3 Hz, 1H), 4.93 (m, 1H), 4.53-4.47 (m, 1H), 4.22-4.18 (m, 2H), 3.60-3.56 (m, 2H), 3.52 (s, 3H), 3.48-3.38 (m, 2H), 3.27 (d, J=6.6 Hz, 1H), 3.08 (s, 1H), 2.15-1.67 (m, 5H), 1.0 (s, 9H); ¹³C NMR (CDCl₃) δ 173.9, 172.1, 170.8, 145.8, 133.6, 129.2, 128.8, 127.4, 123.2, 81.1, 74.5, 72.7, 72.4, 67.6, 59.9, 51.6, 43.2, 41.5, 33.0, 31.8, 29.4, 25.5. HPLC: C-18 Novapac, 4.6X250 mm, 1.5 mL/min; solvent system: 10 - 100% MeCN / H₂O [>20 min gradient]; retention time = 12.2 min.

### EXAMPLE 8: (2R, 3R, 4S, 5R, 6E)-3,4,5-trihydroxy-2-methoxy-8,8-dimethyl-N-[(3S, 6R)-hexahydro-2-oxo-6-(1-oxo-2-[3,4-dichlorophenyl]ethoxy)-2H-azepin-3-yl]non-6-enamide.

Following essentially the procedure of Example 1 g) and using in place of cyclohexanecarbonyl chloride, an approximately equivalent amount of a mixture consisting of: 3,4-dichlorophenylacetic acid , 1-(3-dimethylaminopropyl)-3-ethylcarbodiimide hydrochloride and 4-dimethylaminopyridine; (3*S*, 6*R*)-3-(*tert*-butoxycarbonyl)aminohexahydro-6-(1-oxo-2-[3,4-dichlorophenyl]ethoxy)-2*H*-azepin-2-one is obtained. Employing the compound above in place of compound 1g), and following essentially the procedure of Example 1h), 1i) and the last step of Example 1, the title compound is obtained; mp 132 - 136 °C; ¹H NMR (CDCl₃) δ 8.00 (d, J= 6 Hz, 1H), 7.51 (s, 1H), 7.41 (m, 2H), 7.16 (dd, J=3 Hz and 9 Hz, 1H), 5.77 (d, J=16 Hz, 1H), 5.42 (dd, J= 8 Hz and 16 Hz, 1H), 4.93 (d, J=3 Hz, 1H), 4.55 (q, J=6 Hz, 1H), 4.19 (m, 1H), 3.83 (m, 3H), 3.63 (d, J=6 Hz, 3H), 3.48 (m, 6H), 3.39 (s, 1H), 2.13 (d, J=16 Hz, 1H), 2.00 (m, 2H), 1.77 (q, J=12 Hz, 1H), 1.03 (s, 9H); ¹³C NMR (CDCl₃) δ 169.1, 144.2, 133.5, 131.5, 130.9, 130.8, 129.8, 128.5, 123.4, 81.3, 73.6, 72.2, 71.7, 67.6, 58.4, 50.8, 49.3, 42.0, 32.3, 31.1, 30.7, 28.8, 24.8.

### EXAMPLE 9: (2R, 3R, 4S, 5R, 6E)-3,4,5-trihydroxy-2-methoxy-8,8-dimethyl-N-[(3S, 6R)-hexahydro-2-oxo-6-(1-oxo-2-[4-methoxyphenyl]ethoxy)-2H-azepin-3-yl]non-6-enamide.

### a) Preparation of (3S, 6R)-3-(tert-butoxycarbonyl)aminohexahydro-6-t-butyldimethylsilyloxy-2H-azepin-2-one.

(3*S*, 6*R*)-3-(*tert*-butoxycarbonyl)aminohexahydro-6-hydroxy-2*H*-azepin-2-one (25g, 102 mmol), *tert*-butyldimethylsilyl chloride (23.16g, 153 mmol), and imidazole (10.45g, 153 mmol) are combined with 60 mL of DMF. The reaction is stirred at room temperature overnight. The mixture is diluted with 1 L of water. The resulting mixture is extracted with a 1:1 ( 2 x 200 mL) mixture of ethyl acetate and hexane. All organic layers are combined, washed with brine solution, dried with NaSO₄, and concentrated. The residue is purified by recrystallization with ethyl acetate/hexane to give 28.5g (78%) of (3*S*, 6*R*)-3-(*tert*-butoxycarbonyl)aminohexahydro-6-*tert*-butyldimethylsilyloxy-2*H*-azepin-2-one as a white solid, mp 65 - 66 °C; ¹H NMR (CDCl₃) δ 5.86 (d, J=6 Hz, 1H), 5.58 (t, J=6 Hz, 1H), 4.18 (m, 1H), 3.91 (s, 1H), 3.35(dd, J=6 Hz and 16 Hz, 1H), 3.07 (m, 1H), 1.80 (m, 4H), 1.40 (s, 9H), 0.83 (s, 9H), 0.004 (s, 6H).

### b) Preparation of (3S, 6R)-3-aminohexahydro-6-tert-butyldimethylsilyloxy-2H-azepin-2-one.

(3*S*, 6*R*)-3-(*tert*-butoxycarbonyl)aminohexahydro-6-*tert*-butyldimethylsilyloxy-2H-azepin-2-one (8.0g, 22mmol) is dissolved in 40 mL of CH₂Cl₂ and cooled to -78 ºC. Trimethylsilyl iodide (3.5 mL, 24.5 mmol) is added slowly. The mixture is allowed to react at -78 ºC for 30 min. The reaction is warmed to 0°C and stirred for 15 min. The solution turned yellow. The reaction is quenched with NH₄HCO₃ (3.43g, 44 mmol) dissolved in 30 mL of CH₃OH, and 15 mL water. The mixture is concentrated and chromatographed with 95:5 mixture of CH₂Cl₂ and methanol to yield 5.45g (96%) of (3S, 6R)-3-aminohexahydro-6-*tert*-butyldimethylsilyloxy-2H-azepin-2-one as a white solid: ¹H NMR (CDCl₃) δ 5.61 (s, 1H), 3.88 (s, 1H), 3.42 (d, J=8 Hz, 1H), 3.32(dd, J=6 Hz and 16 Hz, 1H), 3.06 (m, 1H), 1.87 (m, 2H), 1.76 (m, 1H), 1.65 (s, 3H), 0.83 (s, 9H), 0.001 (s, 6H).

### c) Preparation of (2R, 3R, 4S, 5R, 6E)-3,5-(methylethylidene)-3,4,5-trihydroxy-2-methoxy-8,8-dimethyl-N-[(3S, 6R)-hexahydro-2-oxo-6-tert-butyldimethylsilyloxy-2H-azepin-3-yl]non-6-enamide.

(3*S*, 6*R*)-3-aminohexahydro-6-*tert*-butyldimethylsilyloxy-2*H*-azepin-2-one (5.45g, 21 mmol), (6*E*)-6,7,8,9-tetradeoxy-8,8-dimethyl-2-*O*-methyl-3,5-*O*-(1-methylethylidene)-gulo-non-6-enonic acid lactone (3.0 g, 11 mmol), and diisopropylethylamine (4.6 mL, 26 mmol) are combined with 30 mL of isopropanol at room temperature. The mixture is heated to reflux overnight. The mixture is cooled to room temperature and concentrated. The residue is chromatographed with 98:2 mixture of CH₂Cl₂ and methanol to yield 2.53 g (42%) of (2*R*, 3*R*, 4*S*, 5*R*, 6*E*)-3,5-(methylethylidene)-3,4,5-trihydroxy-2-methoxy-8,8-dimethyl-*N*-[(3*S*, 6*R*)-hexahydro-2-oxo-6-*tert*-butyldimethylsilyloxy-2*H*-azepin-3-yl]non-6-enamide (42%) as a white solid: ¹H NMR (CDCl₃) δ 7.53 (d, J=6 Hz, 1H), 5.72 (d, J=16 Hz, 1H), 5.47 (dd, J= 6 Hz and 16 Hz, 1H), 4.47 (m, 1H), 4.22 (d, J=6 Hz, 1H), 4.03 (d, J=8 Hz, 1H), 3.91 (m, 1H), 3.82 (d, J=7 Hz, 1H), 3.48 (d, J=9 Hz, 1H), 3.43 (s, 3H), 3.35 (d, J=6 Hz, 1H), 3.09 (m. 1H), 2.77 (d, J=9 Hz, 1H), 1.83 (m, 2H), 1.77 (m, 2H), 1.41 (d, J=6 Hz, 6H), 0.97 (s, 9H), 0.83 (s, 9H), 0.005 (s, 6H); ¹³C NMR (CDCl₃) δ 172.2, 169.6, 148.3, 145.3, 121.5, 108.8, 99.6, 81.4, 80.5, 79.2, 78.2, 74.4, 73.1, 69.1, 67.9, 65.8, 59.2, 56.4, 51.7, 36.8, 36.5, 33.1, 29.6, 29.4, 19.1.

### d) Preparation of (2R, 3R, 4S, 5R, 6E)-3,5-(methylethylidene)-3,4,5-trihydroxy-2-methoxy-8,8-dimethyl-N-[(3S, 6R)-hexahydro-2-oxo-6-hydroxy-2H-azepin-3-yl]non-6-enamide.

(2*R*, 3*R*, 4*S*, 5*R*, 6*E*)-3,5-(methylethylidene)-3,4,5-trihydroxy-2-methoxy-8,8-dimethyl-*N*-[(3*S*, 6*R*)-hexahydro-2-oxo-6-*tert*-butyldimethylsilyloxy-2*H*-azepin-3-yl]non-6-enamide (2.5 g, 4.6 mmol) is dissolved in 30 mL of THF. 1.0M in THF solution of tetrabutylammonium fluoride (13.8 mL, 14 mmol) is added at room temperature and stirred for 3 hrs. The mixture is concentrated and chromatographed with 95:5 mixture of CH₂Cl₂ and methanol to give 1.8g (91%) of (2*R*, 3*R*, 4*S*, 5*R*, 6*E*)-3,5-(methylethylidene)-3,4,5-trihydroxy-2-methoxy-8,8-dimethyl-*N*-[(3*S*, 6*R*)-hexahydro-2-oxo-6-hydroxy-2*H*-azepin-3-yl]non-6-enamide as a white solid: ¹H NMR (CDCl₃) δ 7.61 (d, J=6 Hz, 1H), 6.45 (t, J=6 Hz, 1H), 5.77 (d, J=6 Hz, 1H), 5.52 (dd, J=6 Hz, and 16 Hz, 1H), 4.56 (m, 1H), 4.28 (d, J=6 Hz, 1H), 4.06 (d, J=8 Hz, 1H), 4.00 (m, 1H), 3.91 (d, J=8 Hz, 1H), 3.54 (m, 1H), 3.47 (s, 3H), 3.35 (m, 2H), 3.08 (d, J=8 Hz, 1H), 2.76 (d, J=6 Hz, 1), 2.02 (m, 2H), 1.83 (m, 2H), 1.45 (s, 6H), 1.03 (s, 9H); ¹³C NMR (CDCl₃) δ 175.1, 169.7, 145.3, 121.5, 99.7, 83.1, 80.6, 74.5, 73.2, 65.8, 64.6, 59.1, 51.8, 45.9, 34.5, 33.1, 29.5, 29.3, 25.1, 19.1, 13.7.

### e) Preparation of (2R, 3R, 4S, 5R, 6E)-3,5-(methylethylidene)-3,4,5-trihydroxy-2-methoxy-8,8-dimethyl-N-[(3S, 6R)-hexahydro-2-oxo-6-(1-oxo-2-[4-methoxyphenyl]ethoxy)-2H-azapan-3-yl]non-6-enamide.

4-Methoxyphenylacetic acid (0.35g, 2.1mmol), 1-(3-dimethylaminopropyl)-3-ethylcarbodiimide hydrochloride (0.42g, 2.1mmol), and DMAP (0.17g, 1.4 mmol) are combined with 30 mL of CH₂Cl₂ and stirred at room temperature for 30 min. (2*R*, 3*R*, 4*S*, 5*R*, 6*E*)-3,5-(methylethylidene)-3,4,5-trihydroxy-2-methoxy-8,8-dimethyl-*N*-[(3*S*, 6*R*)-hexahydro-2-oxo-6-hydroxy-2*H*-azepin-3-yl]non-6-enamide (0.6g, 1.4 mmol) is added to the mixture and stirred overnight at room temperature. The mixture is concentrated. The residue is chromatographed with 98:2 mixture of CH₂Cl₂ and methanol to give 0.644g (80%) of (2*R*, 3*R*, 4*S*, 5*R*, 6*E*)-3,5-(methylethylidene)-3,4,5-trihydroxy-2-methoxy-8,8-dimethy)-*N*-[(3*S*, 6*R*)-hexahydro-2-oxo-6-(1-oxo-2-[4-methoxyphenyl]ethoxy)-2*H*-azapan-3-yl]non-6-enamide as a white solid: ¹H NMR (CDCl₃) δ 7.56 (d, J=6 Hz, 1H), 7.18 (d, J=8 Hz, 2H), 6.86 (d, J=8 Hz, 2H), 5.78 (d, J=16 Hz, 1H), 5.62 (t, J=6 Hz, 1H), 5.52 (dd, J=6 Hz and 16 Hz, 1H), 4.93 (s, 1H), 4.58 (m, 1H), 4.28 (d, J=12 Hz, 1H), 4.06 (d, J=8 Hz, 1H), 3.88 (d, J=7 Hz, 1H), 3.80 (s, 3H), 3.56 (s, 2H), 3.48 (s, 5H), 2.82 (d, J=11 Hz, 1H), 2.05 (m, 3H), 1.71 (s, 2H), 1.42 (d, J=6 Hz, 6H), 1.03 (s, 9H); ¹³C NMR (CDCl₃) δ 174.3, 171.1, 169.7, 158.9, 145.3, 130.2, 125.7, 121.6, 114.2, 99.7, 80.6, 74.5, 73.3, 67.7, 65.8, 59.3, 55.3, 51.7, 43.3, 40.6, 33.1, 31.8, 31.4, 29.4, 25.8, 19.1.

### f) Preparation of the title compound.

30 mL solution of (3:3:2) TFA, THF, and water at 0 ºC is added to a flask containing (2*R*, 3*R*, 4*S*, 5*R*, 6*E*)-3,5-(methylethylidene)-3,4,5-trihydroxy-2-methoxy-8,8-dimethy)-*N*-[(3*S*, 6*R*)-hexahydro-2-oxo-6-(1-oxo-2-[4-methoxyphenyl]ethoxy)-2*H*-azapan-3-yl]non-6-enamide (0.64g, 1.1 mmol). The mixture is allow to react at 0 ºC for 30 min. The mixture is evaporated to dryness under high vacuum. The residue is neutralized with a solution NH₄HCO₃ (1.2g in 20 mL of water). The mixture is evaporated to dryness under high vacuum. The residue is chromatographed with a 95:5 mixture of CH₂Cl₂ and methanol to yield 0.35g (60%) of the title compound as a white solid: ¹H NMR (CDCl₃) δ 7.98 (d, J=6 Hz, 1H), 7.18 (d, J=9 Hz, 2H), 6.87 (d, J=9 Hz, 2H), 5.83 (d, J=16 Hz, 1H), 5.72 (t, J=6 Hz, 1H), 5.42 (dd, J=8 Hz and 16 Hz, 1H), 4.93 (d, J=3 Hz, 1H), 4.53 (m, 1H), 4.23 (t, J=6 Hz, 2H), 3.81 (m, 2H), 3.80 (s, 3H), 3.61 (t, J=5 Hz, 1H), 3.57 (s, 2H), 3.54 (s, 3H), 3.47 (m, 1H), 3.30 (d, J=7 Hz, 1H), 3.12 (s, 1H), 2.16 (d, J=16 Hz, 1H), 1.99 (m, 2H), 1.82 (m, 1H), 1.72 (s, 1H), 1.04 (s, 9H); ¹³C NMR (CDCl₃) δ 173.8, 172.2, 171.1, 158.9, 145.8, 130.2, 125.6, 123.2, 114.2, 81.0, 74.5, 72.8, 72.4, 67.5, 60.0, 55.3, 51.6, 43.2, 40.6, 33.0, 31.8, 31.3, 29.4, 25.6.

### EXAMPLE 10: (2R, 3R, 4S, 5R, 6E)-3,4,5-trihydroxy-2-methoxy-8,8-dimethyl-N-[(3S, 6R)-hexahydro-2-oxo-6-([4-n-decyloxyphenyl]carbonyl)oxy-2H-azepin-3-yl]non-6-enamide.

Following essentially the procedure of Example 9e) and using in place of 4-methoxyphenylacetic acid, an approximately equivalent amount of 4-decyloxybenzoic acid, (2*R*, 3*R*, 4*S*, 5*R*, 6*E*)-3,5-(methylethylidene)-3,4,5-trihydroxy-2-methoxy-8,8-dimethyl-*N*-[(3*S*, 6*R*)-hexahydro-2-oxo-6-([4-*n*-decyloxyphenyl]carbonyl)oxy-2*H*-azepin-3-yl]non-6-enamide is obtained. Employing the compound above in place of compound 9e), and following essentially the procedure of the last step of Example 9, the title compound is obtained as a solid with mp 70 - 74 °C; ¹H NMR (CDCl ) δ 8.06 (d, J=6 Hz, 1H), 7.96 (d, J=9 Hz, 2H), 6.90 (d, J=9 Hz, 2H), 6.05 (t, J=6 Hz, 1H), 5.80 (d, J=15 Hz, 1H), 5.44 (dd, J=7 Hz and 15 Hz, 1H), 5.20 (m, 1H), 4.63 (m, 1H), 4.25 (t, J= 6 Hz, 1H), 4.02 (t, J=6 Hz, 2H), 3.84 (dd, J=7 Hz and 13 Hz, 2H), 3.69 (m, 1H), 3.62 (m, 2H), 3.38 (d, J=5 Hz, 1H), 3.56 (s, 3H), 3.33 (s, 1H), 3.15 (s, 1H), 2.32 (d, J=12 Hz, 1H), 2.13 (t, J=12 Hz, 1H), 2.01 (m, 2H), 1.82 (m, 2H), 1.48 (m, 2H), 1.30 (m, 12H), 1.05 (s, 9H), 0.94 (t, J=6 Hz, 3H); ¹³C NMR (CDCl₃) δ 174.1, 172.1, 165.2, 163.4, 145.7, 131.7, 123.2, 121.6, 114.2, 81.1, 74.5, 72.7, 72.4, 68.3, 67.2, 59.9, 51.7, 43.6, 33.0, 32.1, 31.9, 29.5, 29.4, 29.3, 29.2, 29.0, 25.9, 25.9, 22.6, 14.1.

### EXAMPLE 11: (2R, 3R, 4S, 5R, 6E)-3,4,5-trihydroxy-2-methoxy-8,8-dimethy)-N-[(3S, 6R)-hexahydro-2-oxo-6-(1-oxo-3-phenyl-2-propenoxy)-2H-azepin-3-yl]non-6-enamide.

Following essentially the procedure of Example 1g) and using in place of cyclohexanecarbonyl chloride, an approximately equivalent amount of cinnamoyl chloride, (3*S*, 6*R*)-3-(*tert*-butoxycarbonyl)aminohexahydro-6-(1-oxo-3-phenyl-2-propenoxy)-2*H*-azepin-2-one is obtained. Employing the compound above in place of compound 1g), and following essentially the procedure of Example 1 h), 1 i) and the last step of Example 1, the title compound is obtained as a solid, mp 83 - 85 °C; ¹H NMR (DMSO): δ 8.03 (m, 1H), 7.71 (d, *J* = 16 Hz, 1H), 7.54 (m, 2H), 7.41 (m, 2H), 6.45 (d, *J* = 16Hz, 1H), 5.97 (m, 1H), 5.81 (d, *J*= 16Hz, 1H), 5.45 (dd, *J* = 16 and 8 Hz, 1H), 5.09 (m, 1H), 4.58 (m, 1H), 4.22 (m, 2H), 3.81 (m, 2H), 3.62 (m, 4H), 3.54 (s, 3H), 3.30 (d, *J* = 8Hz, 1H), 3.10 (s, 1H), 2.25 (m, 1H), 2.00 (m, 3H), 1.02 (s, 9H); ¹³C NMR (DMSO): δ 174.10, 172.15, 166.01, 145.91, 145.75, 134.03, 130.70, 129.00, 128.22, 123.20, 117.34, 81.10, 74.50, 72.76, 72.46, 67.29, 59.93, 51.67, 43.51, 33.03, 32.00, 29.43, 25.75.

### EXAMPLE 12: (2R, 3R, 4S, 5R, 6E)-3,4,5-trihydroxy-2-methoxy-8,8-dimethyl-N-[(3S, 6R)-hexahydro-2-oxo-6-([4-n-decylphenyl]carbonyl)oxy-2H-azepin-3-yl]non-6-enamide.

Following essentially the procedure of Example 9e) and using in place of 4-methoxyphenylacetic acid, an approximately equivalent amount of 4-decylbenzoic acid, (2*R*, 3*R*, 4*S*, 5*R*, 6*E*)-3,5-(methylethylidene)-3,4,5-trihydroxy-2-methoxy-8,8-dimethyl-*N*-[(3*S*, 6*R*)-hexahydro-2-oxo-6-([4-*n*-decylphenyl]carbonyl)oxy-2*H*-azepin-3-yl]non-6-enamide is obtained. Employing the compound above in place of compound 9e), and following essentially the procedure of the last step of Example 9, the title compound is obtained: mp 60 - 64 °C; ¹H NMR (CDCl ) δ 8.06 (d, J=6 Hz, 1H), 7.94 (d, J=8 Hz, 2H), 7.26 (d, J=8 Hz, 2H), 6.10 (t, J=7 Hz, 1H), 5.85 (d, J=16 Hz, 1H), 5.45 (dd, J=8 Hz and 16 Hz, 1H), 5.22 (d, J=3 Hz, 1H), 4.64 (dd, J=6 Hz and 8 Hz, 1H), 4.25 (t, J= 6 Hz, 2H), 3.84 (dd, J=6 Hz and 11 Hz, 2H), 3.69 (t, J=7 Hz, 1H), 3.63 (dd, J=5Hz and 8 Hz,2H), 3.56 (s, 3H), 2.68 (t, J=8 Hz, 2H), 2.34 (d, J=13 Hz, 1H), 2.14 (t, J=12 Hz, 1H), 2.04 (t, J=11Hz, 1H), 1.96 (t, J=3 Hz, 1H), 1.63 (m, 2H), 1.32 (m, 18H), 1.82 (m, 2H), 1.05 (s, 9H), 0.90 (t, J=7 Hz, 3H); ¹³C NMR (CDCl₃) δ 174.1, 172.1, 165.5, 149.3, 145.7, 129.7, 128.6, 127.0, 123.2, 81.1, 74.5, 72.7, 72.4, 67.4, 59.9, 51.7, 43.5, 36.0, 33.0, 32.1, 31.9, 31.1, 29.6, 29.5, 29.4, 29.3, 29.2, 25.9, 22.6, 14.1.

### EXAMPLE 13: (2R, 3R, 4S, 5R, 6E)-3,4,5-trihydroxy-2-methoxy-8,8-dimethyl-N-[(3S, 6R)-hexahydro-2-oxo-6-(1-oxo-3-[3-thiophenyl]ethoxy)-2H-azepin-3-yl]non-6-enamide.

Following essentially the procedure of Example 1 g) and using in place of cyclohexanecarbonyl chloride, an approximately equivalent amount of a mixture consisting of: 3-thiopheneacetic acid, 1-(3-dimethylaminopropyl)-3-ethylcarbodiimide hydrochloride and 4-dimethylaminopyridine; (3*S*, 6*R*)-3-(*tert*-butoxycarbonyl)aminohexahydro-6-(1-oxo-3-[3-thiophenyl]ethoxy)-2*H*-azepin-2-one is obtained. Employing the compound above in place of compound 1 g), and following essentially the procedure of Example 1 h), 1 i) and the last step of Example 1, the title compound is obtained: hplc (C-18 Novapac, 4.6X250 mm, 1.5 mL/min) solvent system: 35% MeCN / 65% H₂O [isocratic]; Retention Time = 7.89 min. ¹H NMR (CDCl₃) δ 7.97 (d, J=6 Hz, 1H), 7.30 (m, 1H), 7.14 (m, 1H), 7.00 (m, 1H), 5.83 (m, 1H), 5.81 (d, J=16 Hz, 1H), 5.40 (dd, J=7 Hz and 16Hz, 1H), 4.90 (m, 1H), 4.45 (m, 1H), 4.15 (m, 1H), 3.75 (m, 2H), 3.66 (s, 2H), 3.57 (s, 3H), 3.40 (m, 2H), 3.32 (d, J=7 Hz, 1H), 3.12 (s, 1H), 1.7 (m, 4H), 1.01 (s, 9H); ¹³C NMR (CDCl₃) δ 174.0, 172.1, 170.3, 145.7, 133.1, 126.2, 123.3, 123.1, 81.2, 74.5, 72.8, 72.5, 67.8, 59.9, 51.6, 43.2, 36.0, 33.0, 31.8, 29.5, 25.6.

### EXAMPLE 14: (2R, 3R, 4S, 5R, 6E)-3,4,5-trihydroxy-2-methoxy-8,8-dimethyl-N-[(3S, 6R)-hexahydro-2-oxo-6-(1-oxo-3-[3-indolyl]ethoxy)-2H-azepin-3-yl]non-6-enamide.

Following essentially the procedure of Example 1 g) and using in place of cyclohexanecarbonyl chloride, an approximately equivalent amount of a mixture consisting of: 3-indoleacetic acid, 1-(3-dimethylaminopropyl)-3-ethylcarbodiimide hydrochloride and 4-dimethylaminopyridine; (3*S*, 6*R*)-3-(*tert*-butoxycarbonyl)aminohexahydro-6-(1-oxo-3-[3-indolyl]ethoxy)-2*H*-azepin-2-one is obtained. Employing the compound above in place of compound 1 g), and following essentially the procedure of Example 1 h), 1 i) and the last step of Example 1, the title compound is obtained: hplc (C-18 Novapac, 4.6X250 mm, 1.5 mL/min) solvent system: 35% MeCN / 65% H₂O [isocratic]; retention time = 9.2 min. ¹H NMR (CDCl₃) δ 8.47 (s, 1H), 7.90 (d, J=6 Hz, 1H), 7.55 (d, J=8 Hz, 1H), 7.36 (d, J=8 Hz, 1H), 7.20 - 7.05 (m, 3H), 5.81 (d, J=16 Hz, 1H), 5.75 (m, 1H), 5.37 (dd, J=8 Hz and 16 Hz, 1H), 4.8 (m, 1H), 4.45 - 4.30 (m, 2H), 4.25 - 4.15 (m, 1H), 3.9 - 3.7 (m, 4H), 3.65 - 3.55 (m, 1H), 3.50 (s, 3H), 3.45 - 3.30 (m, 1H), 3.25 - 3.10 (m, 2H), 2.15 - 2.00 (m, 1H), 2.00 - 1.60 (m, 4H), 0.99 (s, 9H); ¹³C NMR (CDCl₃) δ 173.9, 171.9, 145.7, 136.2, 127.1, 123.3, 123.3, 122.3, 119.8, 118.6, 111.5, 108.0, 81.6, 74.4, 72.7, 72.6, 67.5, 59.7, 51.5, 43.1, 33.0, 31.8, 31.6, 29.5, 25.6.

### EXAMPLE 15: Infusion

The compound of Example 1 (15 mg) is dissolved in 98-100 % propylene glycol (1.0 ml). The solution is sterile filtered through a 0.22 microns pore size filter and charged to 1 ml ampoules. The filled ampoules are used for storage and shipment. The filled ampoules are stable for a period of at least 12 months at a temperature of 2 to 8 °C. Prior to intravenous administration, the contents of an ampoule are added to 250 to 1000 ml of a 5 % glucose solution in water-for-injection. The intravenous solution thus formed is stable for a period of 8 hours at room temperature.

Following are the corresponding structures of the compounds of Examples 1-14:

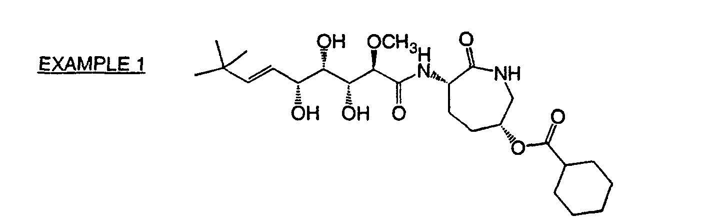

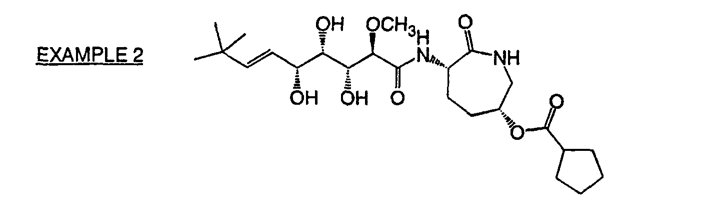

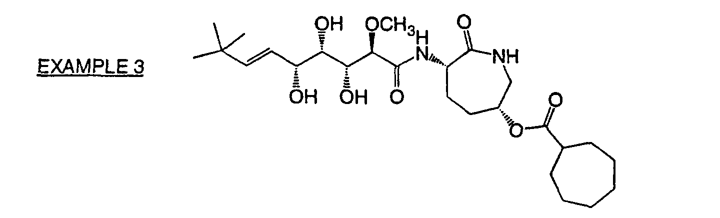

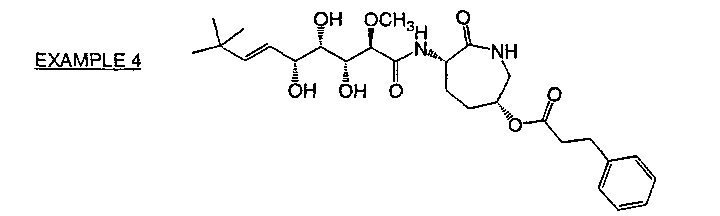

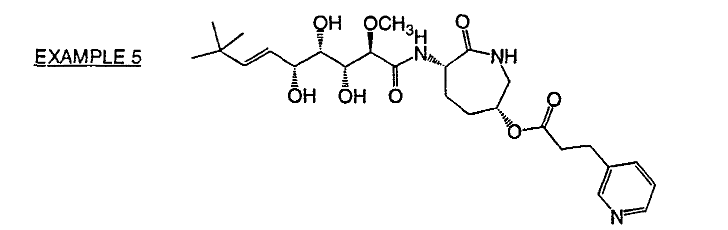

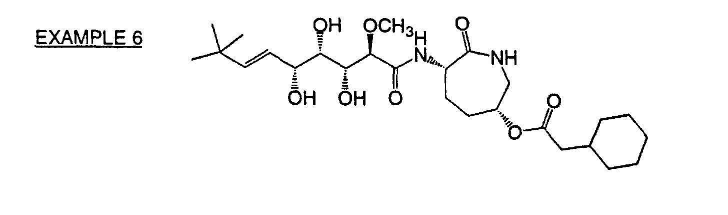

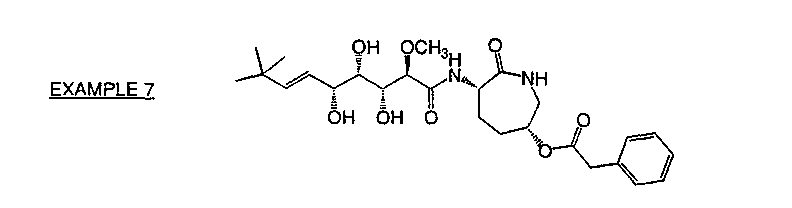

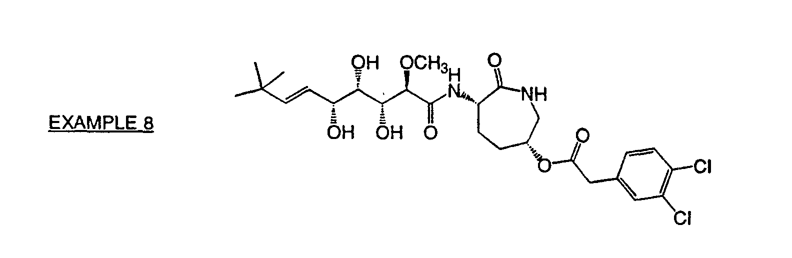

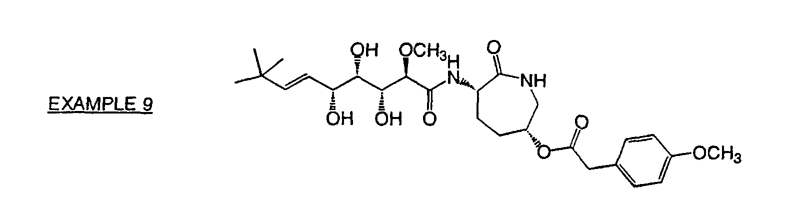

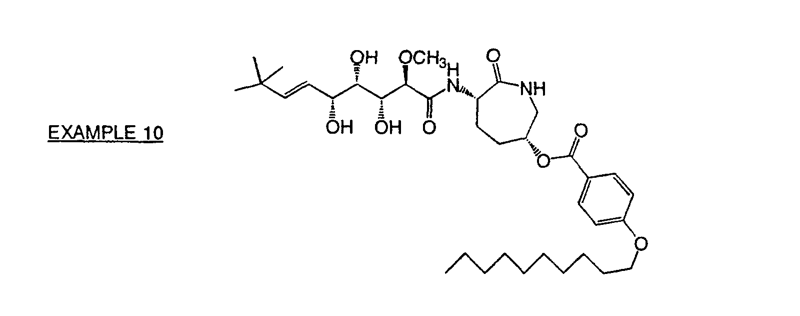

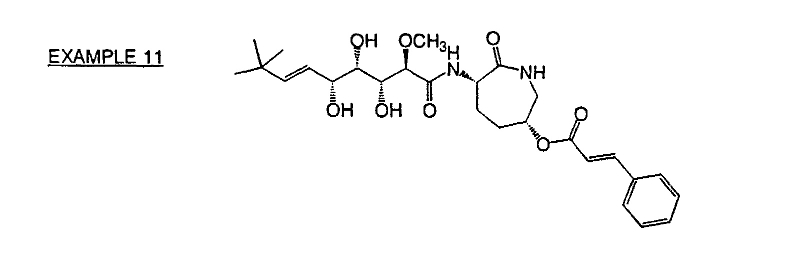

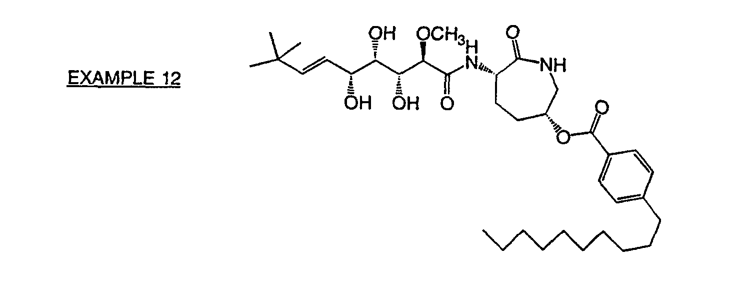

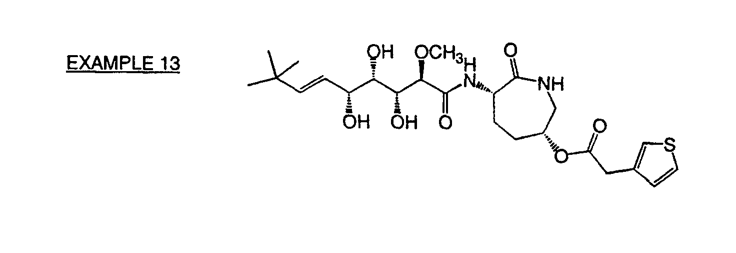

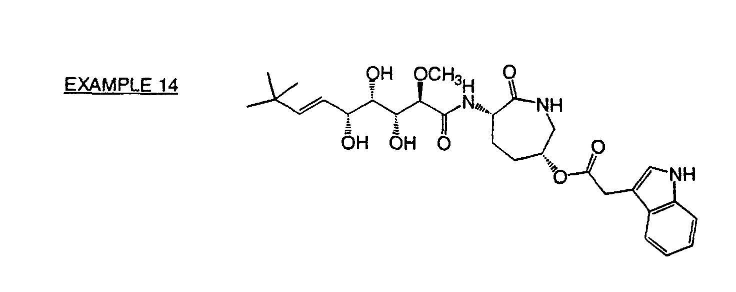

## Claims

1. A compound of formula I: 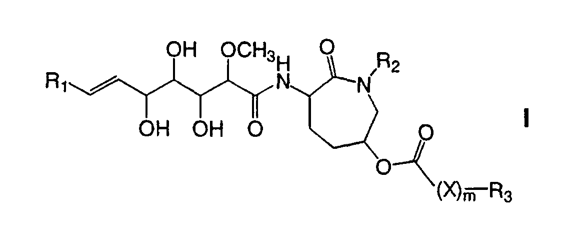 where
R₁ is (C₁₋₆)alkyl or (C₃₋₆)cycloalkyl;
R₂ is hydrogen or (C₁₋₆)alkyl;
X is (C₁₋₁₂) alkylene; (C₂₋₁₂) alkenylene; or (C₂₋₁₂) alkynylene;
m is 0 or 1; and
R₃ is (C₃₋₈)cycloalkyl; or an aromatic ring system selected from II, III, IV and V: 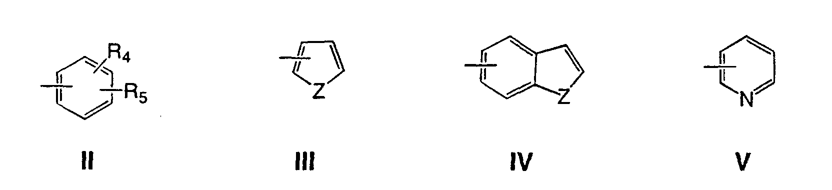
where
R₄ is hydrogen, chloro, or methoxy;
R₅ is hydrogen, chloro, (C₁₋₁₈)alkyl or (C₁₋₁₈)alkoxy; and Z is oxygen, sulfur, N-H, or N-CH₃;
or a pharmaceutically acceptable acid addition salt thereof, where possible.

2. A compound of formula I according to claim 1, where
R₁ is (C₁₋₆) alkyl;
R₂ is hydrogen or (C₁₋₄) alkyl;
X is (C₁₋₆) alkylene or (C₂₋₆) alkenylene;
m is 0 or 1; and
R₃ is (C₃₋₈)cycloalkyl; or an aromatic ring system selected from II, III, IV and V where
R₄ is hydrogen, chloro, or methoxy;
R₅ is hydrogen, chloro, (C₁₋₁₈)alkyl or (C₁₋₁₈)alkoxy; and Z is oxygen, sulfur, N-H, or N-CH₃;
or a pharmaceutically acceptable acid addition salt thereof, where possible.

3. A compound of formula I according to claim 1 or 2 where
R₁ is *i*-propyl or *t*-butyl;
R₂ is hydrogen or methyl;
m is 0 or 1;
X is (C₁₋₆) alkylene; and
R₃ is (C₅₋₇)cycloalkyl; or an aromatic ring system selected from IIa and V: 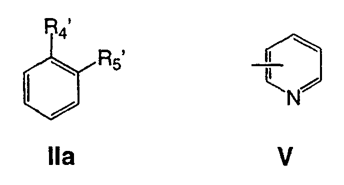
where
R₄' is in the *meta* position and is hydrogen or chloro; and
R₅' is in the *para* position and is hydrogen, chloro, (C₁₋₁₈)alkyl or (C₁₋₁₈)alkoxy;
or a pharmaceutically acceptable acid addition salt thereof, where possible.

4. A compound of formula I according to any one of claims 1-3 where
R₁ is *i*-propyl or *t*-butyl;
R₂ is hydrogen or methyl;
m is 0 or 1;
X is methylene or ethylene; and
R₃ is (C₅₋₇)cycloalkyl, phenyl, 3,4-dichlorophenyl, 4-methoxyphenyl, 4-*n*-decylphenyl, 4-*n*-decyloxyphenyl or 3-pyridyl;
with the proviso that when m is 0, R₃ is (C₅₋₇)cycloalkyl, 4-*n*-decylphenyl or 4-*n*-decyloxyphenyl;
or a pharmaceutically acceptable acid addition salt thereof, where possible.

5. A compound of formula I according to Claim 1, selected from
3,4,5-trihydroxy-2-methoxy-8,8-dimethyl-*N*-[hexahydro-2-oxo-6-(cyclohexylcarbonyl)oxy-2*H*-azepin-3-yl]non-6-enamide, and
3,4,5-trihydroxy-2-methoxy-8,8-dimethyl-*N*-[hexahydro-2-oxo-6-(1-oxo-3-phenylpropoxy)-*2H*-azepin-3-yl]non-6-enamide
or a pharmaceutically acceptable salt thereof.

6. A compound of formula I according to claim 1, selected from
(2*R*, 3*R*, 4*S*, 5*R*, 6*E*)-3,4,5-trihydroxy-2-methoxy-8,8-dimethyl-*N*-[(3*S*, 6*R*)-hexahydro-2-oxo-6-(cyclohexylcarbonyl)oxy 2*H*-azepin-3-yl]non-6-enamide, and
(2*R*, 3*R*, 4*S*, 5*R*, 6*E*)-3,4,5-trihydroxy-2-methoxy-8,8-dimethyl-*N*-[(3*S*, 6*R*)-hexahydro-2-oxo-6-(1-oxo-3-phenylpropoxy-2*H*-azepin-3-yl]non-6-enamide
or a pharmaceutically acceptable salt thereof.

7. A pharmaceutical composition comprising a compound of formula I according to any one of claims 1-6 or a pharmaceutically acceptable salt thereof, where possible, together with a pharmaceutically acceptable carrier.

8. A pharmaceutical composition according to claim 7 comprising a therapeutically effective amount of a compound of formula I which is 3,4,5-trihydroxy-2-methoxy-8,8-dimethyl-*N*-[hexahydro-2-oxo-6-(cyclohexylcarbonyl)oxy-2*H*-azepin-3-yl]non-6-enamide.

9. A pharmaceutical composition according to claim 7 comprising a therapeutically effective amount of a compound of formula I which is 3,4,5-trihydroxy-2-methoxy-8,8-dimethyl-*N*-[hexahydro-2-oxo-6-(1-oxo-3-phenylpropoxy)-2*H*-azepin-3-yl]non-6-enamide.

10. A pharmaceutical composition for the treatment of tumours in warm-blooded animals, including humans, comprising an antitumourally effective dose of a compound of the formula I according to any one of claims 1-6 or a pharmaceutically acceptable salt of such a compound together with a pharmaceutical carrier.

11. The use of a compound of formula I according to any one of claims 1-6 or of a pharmaceutically acceptable salt of such a compound for the preparation of a pharmaceutical composition for use in the chemotherapy of tumours.

12. A process for preparing a caprolactam compound of formula I 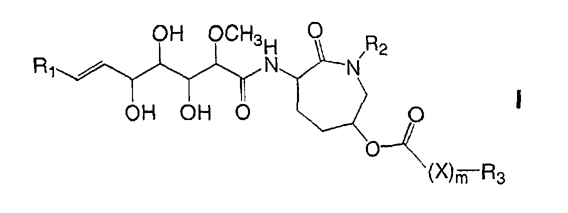 where
R₁ is (C₁₋₆)alkyl or (C₃₋₆)cycloalkyl;
R₂ is hydrogen or (C₁₋₆)alkyl;
X is (C₁₋₁₂) alkylene; (C₂₋₁₂) alkenylene; or (C₂₋₁₂) alkynylene;
m is 0 or 1; and
R₃ is (C₃₋₈)cycloalkyl; or an aromatic ring system selected from II, III, IV and V: 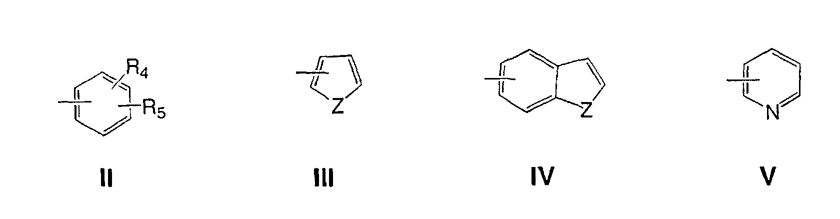 where
R₄ is hydrogen, chloro, or methoxy;
R₅ is hydrogen, chloro, (C₁₋₁₈)alkyl or (C₁₋₁₈)alkoxy; and Z is oxygen, sulfur, N-H, or N-CH₃;
or a pharmaceutically acceptable acid addition salt thereof, where possible,
which process comprises, in a first step, acylating an aminocaprolactam compound of formula VI 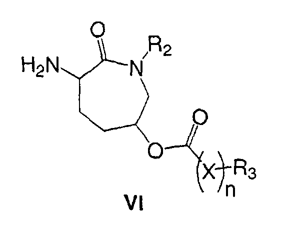 with a lactone compound of formula VII 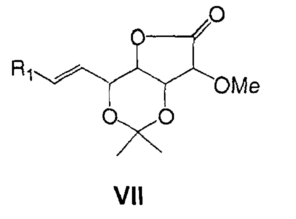 in the presence of a polar, organic solvent to obtain a diamide compound of formula VIII 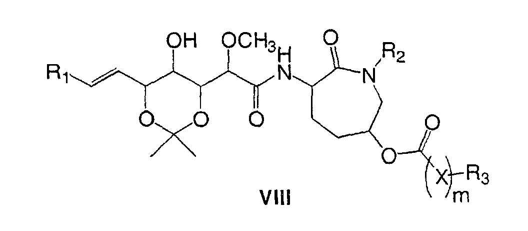 where each of R₁, R₂, X, m and R₃ is as defined in claim 1 and, in a second step, hydrolyzing the diamide compound obtained in the first step by dissolving it in a mixture of solvents to obtain the desired caprolactam compound of formula I.

13. A process according to claim 12 wherein the acylation step is carried out in the presence of isopropanol at a temperature slightly below or at the reflux temperature of the isopropanol.

## Patentansprüche

1. Verbindung der Formel I 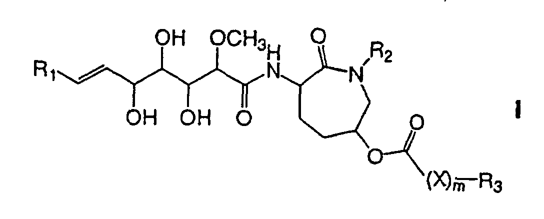
worin R₁ für C₁-C₆ Alkyl oder C₃-C₆ Cycloalkyl steht,
R₂ für Wasserstoff oder C₁-C₆ Alkyl steht,
X für C₁-C₁₂ Alkylen, C₂-C₁₂ Alkenylen oder C₂-C₁₂ Alkinylen steht,
m für 0 oder 1 steht und
R₃ für C₃-C₈ Cycloalkyl oder ein aromatisches Ringsystem steht, ausgewählt aus II, III, IV und V 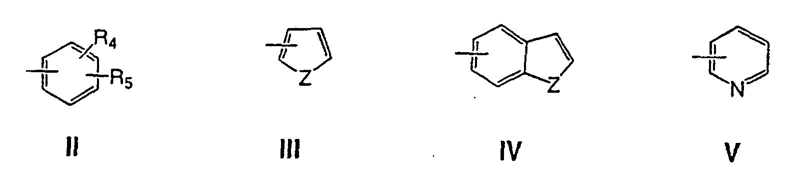
worin
R₄ für Wasserstoff, Chlor oder Methoxy steht,
R₅ für Wasserstoff, Chlor, C₁-C₁₈ Alkyl oder C₁-C₁₈ Alkoxy steht und Z für Sauerstoff, Schwefel, N-H oder N-CH₃ steht,
oder ein pharmazeutisch annehmbares Säureadditionssalz hiervon, wo dies möglich ist.

2. Verbindung der Formel I nach Anspruch 1, worin
R₁ für C₁-C₆ Alkyl steht,
R₂ für Wasserstoff oder C₁-C₄ Alkyl steht,
X für C₁-C₆ Alkylen oder C₂-C₆ Alkenylen steht,
m für 0 oder 1 steht, und
R₃ für C₃-C₈ Cycloalkyl oder ein aromatisches Ringsystem steht, ausgewählt aus II, III, IV und V, worin
R₄ für Wasserstoff, Chlor oder Methoxy steht,
R₅ für Wasserstoff, Chlor, C₁-C₁₈ Alkyl oder C₁-C₁₈ Alkoxy steht und Z für Sauerstoff, Schwefel, N-H oder N-CH₃ steht,
oder ein pharmazeutisch annehmbares Säureadditionssalz hiervon, wo dies möglich ist.

3. Verbindung der Formel I nach Anspruch 1 oder 2, worin
R₁ für i-Propyl oder t-Butyl steht,
R₂ für Wasserstoff oder Methyl steht,
m für 0 oder 1 steht,
X für C₁-C₆ Alkylen steht, und
R₃ für C₅-C₇ Cycloalkyl oder ein aromatisches Ringsystem steht, ausgewählt aus IIa und V 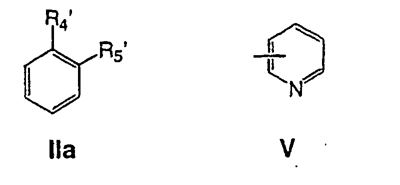
worin
R₄' in der meta Position steht und für Wasserstoff oder Chlor steht, und
R₅' in der para Position steht und für Wasserstoff, Chlor, C₁-C₁₈ Alkyl oder C₁-C₁₈ Alkoxy steht,
oder ein pharmazeutisch annehmbares Säureadditionssalz hiervon, wo dies möglich ist.

4. Verbindung der Formel I nach einem der Ansprüche 1 bis 3, worin
R₁ für i-Propyl oder t-Butyl steht,
R₂ für Wasserstoff oder Methyl steht,
m für 0 oder 1 steht,
X für Methylen oder Ethylen steht, und
R₃ für C₅-C₇ Cycloalkyl, Phenyl, 3,4-Dichlorphenyl, 4-Methoxyphenyl, 4-n-Decylphenyl, 4-n-Decyloxyphenyl oder 3-Pyridyl steht,
mit der Maßgabe, dass wenn m für 0 steht, R₃ dann für C₅-C₇ Cycloalkyl, 4-n-Decylphenyl oder 4-n-Decyloxyphenyl steht,
oder ein pharmazeutisch annehmbares Säureadditionssalz hiervon, wo dies möglich ist.

5. Verbindung der Formel I nach Anspruch 1, ausgewählt aus
3,4,5-Trihydroxy-2-methoxy-8,8-dimethyl-N-[hexahydro-2-oxo-6-(cyclohexylcarbonyl)oxy-2H-azepin-3-yl]non-6-enamid und
3,4,5-Trihydroxy-2-methoxy-8,8-dimethyl-N-[hexahydro-2-oxo-6-(1-oxo-3-phenylpropoxy)-2H-azepin-3-yl]non-6-enamid,
oder ein pharmazeutisch annehmbares Salz hiervon.

6. Verbindung der Formel I nach Anspruch 1, ausgewählt aus
(2R,3R,4S,5R,6E)-3,4,5-Trihydroxy-2-methoxy-8,8-dimethyl-N-[(3S,6R)-hexahydro-2-oxo-6-(cyclohexylcarbonyl)oxy-2H-azepin-3-yl]non-6-enamid, und
(2R,3R,4S,5R,6E)-3,4,5-trihydroxy-2-methoxy-8,8-dimethyl-N-[(3S,6R)-hexahydro-2-oxo-6-(1-oxo-3-phenylpropoxy-2H-azepin-3-yl]non-6-enamid
oder ein pharmazeutisch annehmbares Salz hiervon.

7. Pharmazeutische Zusammensetzung, die eine Verbindung der Formel I nach einem der Ansprüche 1 bis 6 oder ein pharmazeutisch annehmbares Salz hiervon, wo dies möglich ist, zusammen mit einem pharmazeutisch annehmbaren Träger umfasst.

8. Pharmazeutische Zusammensetzung nach Anspruch 7, die eine therapeutisch wirksame Menge einer Verbindung der Formel I umfasst, die 3,4,5-Trihydroxy-2-methoxy-8,8-dimethyl-N-[hexahydro-2-oxo-6-(cyclohexylcarbonyl)-oxy-2H-azepin-3-yl]non-6-enamid ist.

9. Pharmazeutische Zusammensetzung nach Anspruch 7, die eine therapeutisch wirksame Menge einer Verbindung der Formel I umfasst, die 3,4,5-Trihydroxy-2-methoxy-8,8-dimethyl-N-[hexahydro-2-oxo-6-(1-oxo-3-phenylpropoxy)-2H-azepin-3-yl]non-6-enamid ist.

10. Pharmazeutische Zusammensetzung zur Behandlung von Tumoren bei Warmblütern, einschließlich dem Menschen, die eine wirksame Antitumordosis einer Verbindung der Formel I nach einem der Ansprüche 1 bis 6 oder ein pharmazeutisch annehmbares Salz einer solchen Verbindung zusammen mit einem pharmazeutischen Träger umfasst.

11. Verwendung einer Verbindung der Formel I nach einem der Ansprüche 1 bis 6 oder eines pharmazeutisch annehmbaren Salzes einer solchen Verbindung zur Herstellung einer pharmazeutischen Zusammensetzung zur Verwendung bei der Chemotherapie von Tumoren.

12. Verfahren zur Herstellung einer Caprolactamverbindung der Formel I 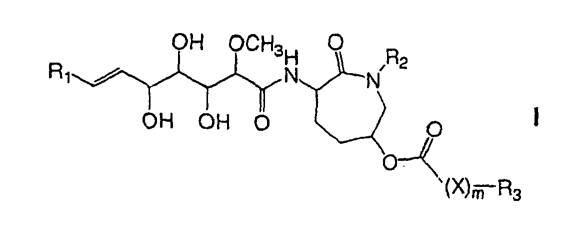 worin
R₁ für C₁-C₆ Alkyl oder C₃-C₆ Cycloalkyl steht,
R₂ für Wasserstoff oder C₁-C₆ Alkyl steht,
X für C₁-C₁₂ Alkylen, C₂-C₁₂ Alkenylen oder C₂-C₁₂ Alkinylen steht,
m für 0 oder 1 steht, und
R₃ für C₃-C₈ Cycloalkyl oder ein aromatisches Ringsystem steht, ausgewählt aus II, III, IV und V 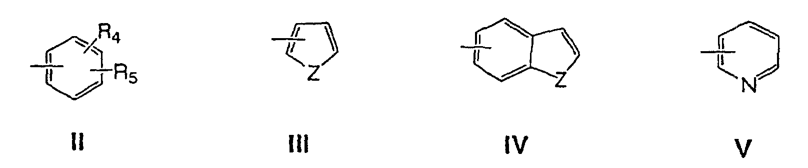
worin
R₄ für Wasserstoff, Chlor oder Methoxy steht,
R₅ für Wasserstoff, Chlor, C₁-C₁₈ Alkyl oder C₁-C₁₈ Alkoxy steht und Z für Sauerstoff, Schwefel, N-H oder N-CH₃ steht,
oder ein pharmazeutisch annehmbares Säureadditionssalz hiervon, wo dies möglich ist,
wobei das Verfahren in einem ersten Schritt die Acylierung einer Aminocaprolactamverbindung der Formel VI 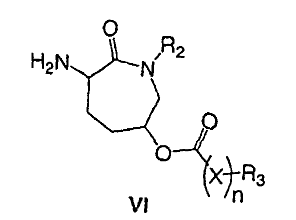 mit einer Lactonverbindung der Formel VII 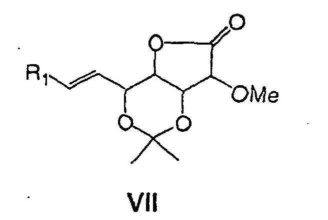 in Gegenwart eines polaren, organischen Lösemittels unter Bildung einer Diamidverbindung der Formel VIII 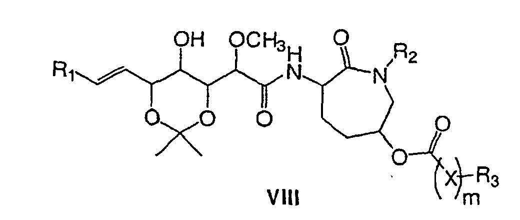 worin jeweils R₁, R₂, X, m und R₃ wie in Anspruch 1 definiert sind und in einem zweiten Schritt die Hydrolyse der im ersten Schritt erhaltenen Diamidverbindung durch Lösen in einem Lösemittelgemisch umfasst, um die gewünschte Caprolactamverbindung der Formel I zu erhalten.

13. Verfahren nach Anspruch 12, worin der Acylierungsschritt in Gegenwart von Isopropanol bei einer Temperatur etwas unterhalb der Rückflusstemperatur von Isopropanol ausgeführt wird.

## Revendications

1. Composé de formule 1 : 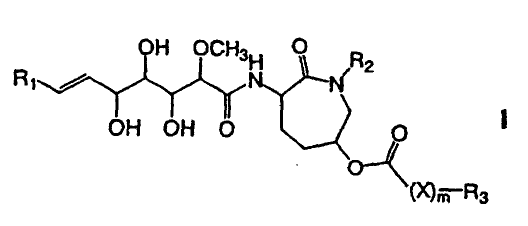 dans laquelle :
R₁ est un groupe alkyle en C₁ à C₆ ou cycloalkyle en C₃ à C₆ ;
R₂ représente l'hydrogène ou un groupe alkyle en C₁ à C₆ ;
X est un groupe alkylène en C₁ à C₁₂, alcénylène en C₂ à C₁₂ ou alcynylène en C₂ à C₁₂ ;
m vaut 0 ou 1 ; et
R₃ est un groupe cycloalkyle en C₃ à C₈ ; ou un système de noyau aromatique choisi entre II, III, IV et V : 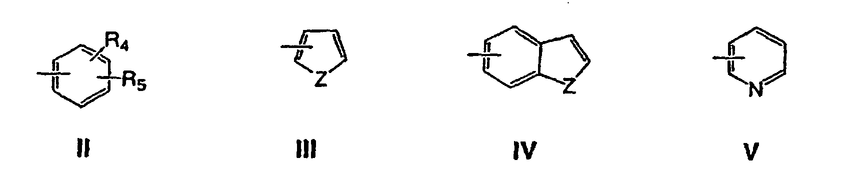
où :
R₄ représente l'hydrogène, un groupe chloro ou méthoxy ;
R₅ représente l'hydrogène, un groupe chloro, alkyle en C₁ à C₁₈ ou alcoxy en C₁ à C₁₈ ; et Z représente l'oxygène, le soufre, N-H ou N-CH₃ ;
ou un de ses sels d'addition d'acides pharmaceutiquement acceptables, lorsque cela est possible.

2. Composé de formule I suivant la revendication 1, dans laquelle :
R₁ est un groupe alkyle en C₁ à C₆ ;
R₂ représente l'hydrogène ou un groupe alkyle en C₁ à C₄ ;
X est un groupe alkylène en C₁ à C₆ ou alcénylène en C₂ à C₆ ;
m vaut 0 ou 1 ; et
R₃ est un groupe cycloalkyle en C₃ à C₈ ; ou un système de noyau aromatique choisi entre II, III, IV et V, où
R₄ représente l'hydrogène, un groupe chloro ou méthoxy ;
R₅ représente l'hydrogène, un groupe chloro, alkyle en C₁ à C₁₈ ou alcoxy en C₁ à C₁₈ ; et Z représente l'oxygène, le soufre, N-H ou N-CH₃ ;
ou un de ses sels d'addition d'acides pharmaceutiquement acceptables, lorsque cela est possible.

3. Composé de formule I suivant la revendication 1 ou 2, dans laquelle
R₁ est un groupe i-propyle ou t-butyle ;
R₂ représente l'hydrogène ou un groupe méthyle ;
m vaut 0 ou 1 ;
X est un groupe alkylène en C₁ à C₆ ; et
R₃ est un groupe cycloalkyle en C₅ à C₇ ; ou un système de noyau aromatique choisi entre IIa et V : 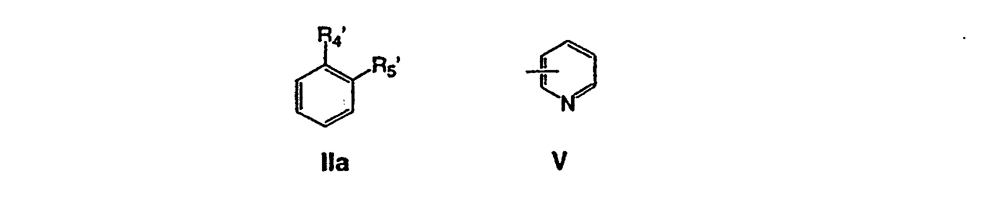
où :
R₄' est en position méta et représente l'hydrogène ou un groupe chloro ; et
R₅' est en position para et représente l'hydrogène, un groupe chloro, alkyle en C₁ à C₁₈ ou alcoxy en C₁ à C₁₈ ;
ou un de ses sels d'addition d'acides pharmaceutiquement acceptables, lorsque cela est possible.

4. Composé de formule I suivant l'une quelconque des revendications 1 à 3, dans laquelle :
R₁ est un groupe i-propyle ou t-butyle ;
R₂ représente l'hydrogène ou un groupe méthyle ;
m vaut 0 ou 1 ;
X est un groupe méthylène ou éthylène ; et
R₃ est un groupe cycloalkyle en C₅ à C₇, phényle, 3,4-dichlorophényle, 4-méthoxyphényle, 4-n-décylphényle, 4-n-décyloxyphényle ou 3-pyridyle ;
sous réserve que lorsque m vaut 0, R₃ soit un groupe cycloalkyle en C₅ à C₇, 4-n-décylphényle ou 4-n-décyloxyphényle ;
ou un de ses sels d'addition d'acides pharmaceutiquement acceptables, lorsque cela est possible.

5. Composé de formule I suivant la revendication 1, choisi entre
le 3,4,5-trihydroxy-2-méthoxy-8,8-diméthyl-N-[hexahydro-2-oxo-6-(cyclohexylcarbonyl)oxy-2H-azépin-3-yl]non-6-énamide, et
le 3,4,5-trihydroxy-2-méthoxy-8,8-diméthyl-N-[hexahydro-2-oxo-6-(1-oxo-3-phénylpropoxy)-2H-azépin-3-yl]non-6-énamide,
ou un de ses sels pharmaceutiquement acceptables.

6. Composé de formule I suivant la revendication 1, choisi entre
le (2R,3R,4S,5R,6E)-3,4,5-trihydroxy-2-méthoxy-8,8-diméthyl-N-[(3S,6R)-hexahydro-2-oxo-6-(cyclohexylcarbonyl)oxy-2H-azépin-3-yl]non-6-énamide, et
le (2R,3R,4S,5R,6E)-3,4,5-trihydroxy-2-méthoxy-8,8-diméthyl-N-[(3S,6R)-hexahydro-2-oxo-6-(1-oxo-3-phénylpropoxy-2H-azépin-3-yl]non-6-énamide,
ou un de ses sels pharmaceutiquement acceptables.

7. Composition pharmaceutique comprenant un composé de formule I suivant l'une quelconque des revendications 1 à 6, ou un de ses sels pharmaceutiquement acceptables, lorsque cela est possible, conjointement avec un véhicule pharmaceutiquement acceptable.

8. Composition pharmaceutique suivant la revendication 7, comprenant une quantité thérapeutiquement efficace d'un composé de formule I, qui est le 3,4,5-trihydroxy-2-méthoxy-8,8-diméthyl-N-[hexahydro-2-oxo-6-(cyclohexylcarbonyl)oxy-2H-azépin-3-yl]non-6-énamide.

9. Composition pharmaceutique suivant la revendication 7, comprenant une quantité thérapeutiquement efficace d'un composé de formule I, qui est le 3,4,5-trihydroxy-2-méthoxy-8,8-diméthyl-N-[hexahydro-2-oxo-6-(1-oxo-3-phénylpropoxy)-2H-azépin-3-yl]non-6-énamide.

10. Composition pharmaceutique pour le traitement de tumeurs chez des animaux homéothermes, y compris l'homme, comprenant une dose efficace du point de vue antitumoral d'un composé de formule I suivant l'une quelconque des revendications 1 à 6, ou d'un sel pharmaceutiquement acceptable d'un tel composé, conjointement avec un véhicule pharmaceutique.

11. Utilisation d'un composé de formule I suivant l'une quelconque des revendications 1 à 6, ou d'un sel pharmaceutiquement acceptable d'un tel composé pour la préparation d'une composition pharmaceutique à utiliser dans la chimiothérapie des tumeurs.

12. Procédé de préparation d'un composé de caprolactame de formule 1 : 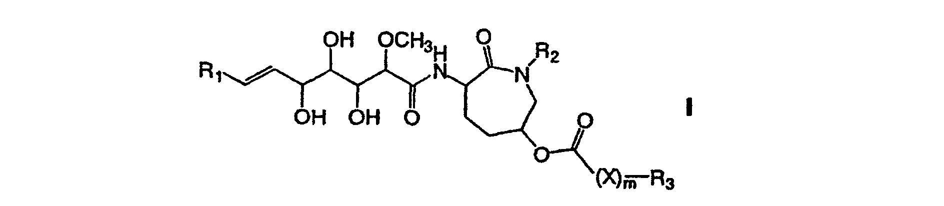 dans laquelle :
R₁ est un groupe alkyle en C₁ à C₆ ou cycloalkyle en C₃ à C₆ ;
R₂ représente l'hydrogène ou un groupe alkyle en C₁ à C₆ ;
X est un groupe alkylène en C₁ à C₁₂, alcénylène en C₂ à C₁₂ ou alcynylène en C₂ à C₁₂ ;
m vaut 0 ou 1 ; et
R₃ est un groupe cycloalkyle en C₃ à C₈ ; ou un système de noyau aromatique choisi entre II, III, IV et V : 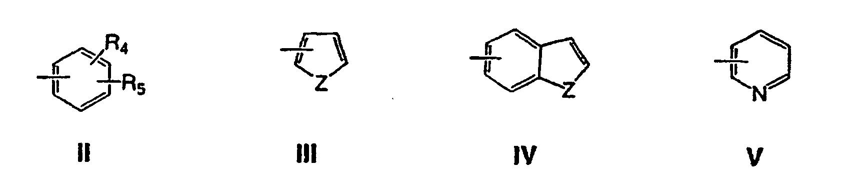
où :
R₄ représente l'hydrogène, un groupe chloro ou méthoxy ;
R₅ représente l'hydrogène, un groupe chloro, alkyle en C₁ à C₁₈ ou alcoxy en C₁ à C₁₈ ; et Z représente l'oxygène, le soufre, N-H ou N-CH₃ ;
ou d'un de ses sels d'addition d'acide pharmaceutiquement acceptables, lorsque cela est possible,
ledit procédé comprenant, dans une première étape, l'acylation d'un composé d'aminocaprolactame de formule VI : 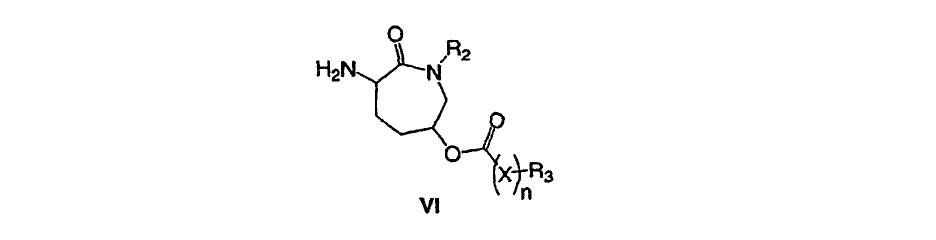 avec un composé de lactone de formule VII : 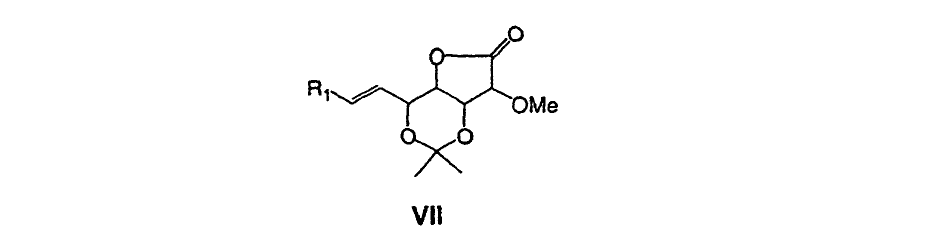 en présence d'un solvant organique polaire pour obtenir un composé de diamide de formule VIII : 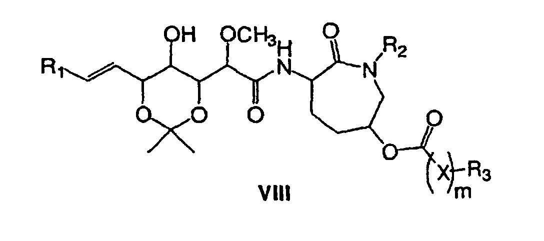 dans laquelle chacun des radicaux R₁, R₂, X et R₃, et m, sont tels que définis dans la revendication 1 et, dans une seconde étape, l'hydrolyse du composé de diamide obtenu dans la première étape en le dissolvant dans un mélange de solvants pour obtenir le composé de caprolactame désiré de formule I.

13. Procédé suivant la revendication 12, dans lequel l'étape d'acylation est conduite en présence d'isopropanol à une température légèrement inférieure ou égale à la température de reflux de l'isopropanol.
